# EUROPEAN PATENT SPECIFICATION

(11) **EP 4 311 402 B1**
(45) Date of publication and mention of the grant of the patent: **22.05.2024**
(21) Application number: 22735124.4
(22) Date of filing: 15.06.2022
(51) Int. Cl.: C12M 1/00, G01N 1/40

(54) **CELL RECOVERY METHOD AND DEVICE**
VERFAHREN UND VORRICHTUNG ZUR ZELLRÜCKGEWINNUNG
PROCÉDÉ ET DISPOSITIF DE RÉCUPÉRATION DE CELLULES

(43) Date of publication of application: 31.01.2024
(73) Proprietor: Angle Europe Limited, Guildford, GU2 7AF (GB)
(72) Inventor: IZMAILOV, Alexandre, Ontario, M8X 1H4 (CA); PAK, Brian, Ontario, L4E 0T3 (CA); SEN, Tomoyuki, Toronto, M5V 0J2 (CA); SMITH, Paul, Acton, L7J 2L7 (CA)
(74) Representative: HGF
(86) International application number: PCT/EP2022/066395
(87) International publication number: WO 2023/241796

(56) References cited:
- WO-A2-2007/008446
- US-A1- 2003 175 850
- US-A1- 2008 214 378

## Description

### FIELD

The present disclosure relates to a cell recovery method and device for the efficient sedimentation and retention of cells from liquid samples onto a solid support with low cell loss and low impact on cell morphology.

### BACKGROUND

There are many methods/devices used to isolate cells from liquid samples. Many involve the use of cytocentrifugation which accelerates cell sedimentation towards a desired collection point. Drawbacks to many of these methods/devices include significant cell loss and altered cell morphology which may be detrimental for subsequent analysis.

In clinical applications, a variety of different fluid specimens are routinely analyzed for the presence and characterization of cells. These include blood, urine, cerebrospinal fluid, synovial fluid and ascites. In blood, a population of so-called "circulating rare cells" have been identified in some patients representing cells that have disseminated from their tissue of origin. Amongst the most sought-after type of circulating rare cells are circulating tumour cells (CTC), which, if present, may provide important information on disease status and prognosis.

The efficient recovery of cells in samples containing rare cells is significant as even a moderate cell loss during sample processing may result in incorrect enumeration or a false negative result where a sample containing rare cells may be erroneously designated as having none. Many technologies have recently emerged which enrich circulating rare cells from blood.

Various methods are used for extracting rare cells from patient samples (cell recovery) resulting typically in a fluid sample containing cells of interest in combination with other cells (e.g., white blood cells). Harvested cells are important for researchers and in most of the cases microscopic study and consecutive molecular analysis of these cells is conducted. In order to make this analysis possible the cells present in the sample should be sedimented (deposited) onto a surface of a solid support allowing microscopy of these cells. Typically, the harvested cells are sedimented onto microscopy slides, which can be glass or plastic slides. One of the fundamental requirements for this process is to prevent cell losses. This becomes especially important when the user is working with rare cells: in this case the sample may contain very few of such cells and loss of even small number of cells may make the consecutive analysis unreliable or impossible. The second major requirement is that the process of cell retention should cause minimal morphological change of the cells to aid in cytological analysis.

For example, U.S. Patent No. 7,575,719 discloses a fluid sample chamber that can be sealed onto a microscope slide surface. This means of sealing can be used in conjunction with a filter card placed or ultrasonic welded between the microscope surface and sample chamber. This method of fluid removal poses the risk of unintentional loss of sample to the surrounding absorbent filter card medium.

U.S. Patent No. 7,628,955 discloses a chamber for fluid samples that can be sealed onto the surface of a microscope slide through the use of integrally molded elastomeric gaskets and oil film components. This device does not provide a means of removing liquid in a controlled manner to prevent the removal of sedimented cells from the slide.

U.S. Patent No. 4,696,743 discloses a filter card placed between a sample chamber and deposit-receiving surface such as a microscope slide. Solids present within the fluid suspension are centrifuged to the slide surface while the surrounding fluids are drawn into the adjacent absorbent medium. This device does not address the issue of cell loss by means of separating the fluid removal step from the centrifugation/ sedimentation step.

U.S. Patent Publication No. 2003/0175850 discloses a method of enriching cells of interest from a suspension by utilizing biotin conjugated antibodies to selectively label cells then bind them to a slide surface coated with an avidin/ streptavidin substrate. An absorptive medium wicks off any unbound materials or fluids to leave a dried slide containing samples of interest. This device does not account for the turbulence and sheer stresses resulting from the lateral flow of liquid into the medium at an uncontrolled rate. While this grants the ability to remove unbound cells in suspension, it also leaves the risk of fortuitously removing slide-bound target cells.

U.S. Patent No. 5,419,279 discloses a flanged hollow tube for sample deposition that engages with a corresponding base to securely seal the tube onto a microscope slide for staining purposes. The bottom of the tube features a groove such that an O-ring may be fitted between interface of the slide and tube to prevent leakage. This apparatus is reliant upon the binding forces between a functionalized slide surface and cell rather than centrifugal forces in addition to functionalization to encourage the capture of target cells and further does not address the removal of supernatant throughout the cell sedimentation/ binding steps which may present avenues for cell loss due to the disturbance of surrounding liquid medium.

U.S. Patent No. 5,480,484 discloses a fluidic chamber sealed centrally (relative to the microscope slide) via an elastomeric gasket and metal clips onto a supportive backing plate. The number of fluidic chambers can be changed to accommodate one or multiple samples albeit with a change in allowable maximum sample volume. The assembled centrifugation device free floats within the specified rotor and deposits cells from suspension onto the slide surface upon the application of centrifugal force. This device does not account for a convenient and controlled method of supernatant removal. Rather, the chamber has a relatively narrow opening which does not lend itself to the removal of fluid.

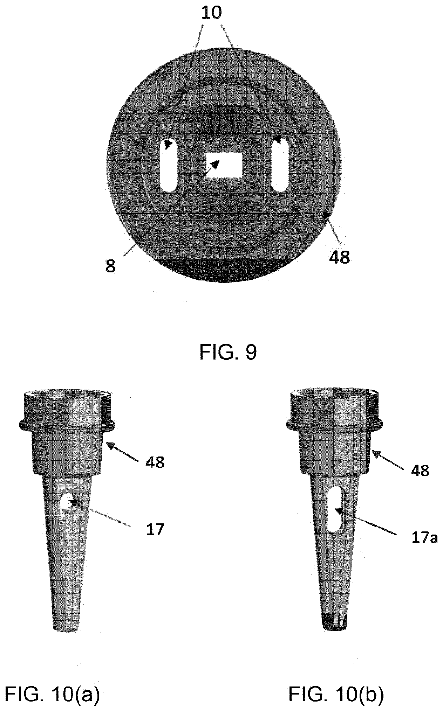

U.S. Patent No. 4,576,110 discloses a rotor chamber designed to deposit a cell suspension onto a microscope slide under the application of centrifugal force. Concurrently, an absorptive plug (shown in Figure 9 of the patent) is placed into a separate compartment within the rotor that protrudes into the same chamber such that the tip of the plug is touching the slide surface. As centrifugal force is applied to the rotor, particles suspended in the liquid are deposited onto the slide surface. According to this patent as the centrifugal force is high enough to overcome the capillary force, the absorbent plug does not draw up the supernatant until after the rotor slows. On the other hand, the absorbing element **3** is introduced into the system prior to centrifugation and absorption starts immediately and lasts until centrifugation forces exceed capillary forces. This potentially leads to loss of the cells present in the solution. While the concept of separating the processes of cell sedimentation and supernatant removal into mutually exclusive steps is similar to our device, there are key differences in the approach to fluid absorption that distinguish the two designs. One of the main features of the prior art is that the absorbent plug protrudes into the same chamber that the particle-containing fluid is deposited into. While capillary action may not act upon the fluid during centrifugation, there is still a possibility of losing cells within the absorbent plug by mere physical entanglement of the cells within the porous material thus leading to cell loss. In addition, the absorbent plug is noted as being in physical contact with the deposition surface when placed within the rotor. The close proximity and lack of control in flow rate of fluid into the absorbent material can result in the unintended absorption of cells that are weakly adhered to the deposition surface.

U.S. Patent No. 4,344,562 discloses a device utilizing a conventional glass slide which can be locked into a housing by means of a holder. Part of the housing is a reservoir in which a fluid sample may be received, the reservoir being closed off at its bottom by the glass slide when the glass slide has been locked into the housing. The patent is aimed to centrifugation of small fluid samples to take place without any loss of cells, enhancing accuracy for purposes of medical diagnosis and treatment. A rubber O-ring is used to provide a fluid tight seal between the components.

US2008/0214378A1 discloses a cytocentrifuge sample chamber that includes a sample receiving well to receive and hold a sample to be centrifuged and a sedimentation chamber adjacent a microscope slide upon which cells and/or other sediment material in the sample is to be deposited. A passage, such as a spillway, connects the sample receiving well and the sedimentation chamber, so that sample flows from the sample well under the influence of centrifugal force as centrifugation begins into the sedimentation chamber where the sample is forced by centrifugal force against the microscope slide leaving a vacant space at the inner end of the sedimentation chamber. Sedimentation of the sample takes place against the microscope slide. When centrifugation ends, the liquid components of the sample flow back into the vacant space in the sedimentation chamber and can be removed from the vacant space at the inner end of the sedimentation chamber through a removal chamber that is adjacent to, and connected with, the vacant space.

### SUMMARY

The present disclosure provides a cell recovery device for sedimentation and retention of targeted cells from a fluid sample as defined in claim 1. The device includes a base configured to releasably hold a solid support with the solid support configured to receive cells on a top surface of the solid support. The device includes a fluid chamber having a first opening with a circumferential gasket surrounding the bottom opening with the base configured to releasably hold the fluid chamber with the gasket bearing against a top surface of the solid support to form a liquid tight seal between the top surface and the solid support, the fluid chamber having a second opening for receiving a liquid sample containing the targeted cells being harvested. The gasket defines an area of selected size into which the targeted cells deposit onto the top surface of the solid support. The device includes a first removable cap configured to close the second opening during centrifugation. The device also includes a fluid absorbing element and an enclosure configured to receive the absorbing element and the fluid chamber is configured to receive the enclosure post centrifugation. The enclosure has a bottom opening through which the fluid from the fluid chamber can be absorbed by the absorbing element when it is introduced into the fluid chamber post centrifugation. The fluid chamber includes a second removable cap configured to close the second opening in place of the first removable cap and to prevent positioning a bottom tip of the absorbing element to a distance from the solid support shorter than a predefined distance. The cell harvesting device is configured to be received and releasably held in a centrifuge.

The fluid absorbing element may have a cross-sectional area, a distribution along an axis of the absorbing element and a porosity in a range to provide control of a rate of absorption of the fluid and the tip of the fluid absorbing element located at the distance from the surface of the solid support such that the targeted cells settled on the solid support are not detached from the surface of the solid support by the flow of the fluid being absorbed by the absorbing element.

The device absorbing element may have a porosity in a range from about 1 about 100 microns, alternatively, the porosity may be in a range from about 5 to about 50 microns, or alternatively the porosity may be in a range from about 10 to about 20 microns. The predefined distance of the tip of the absorbing element above the surface of solid substrate is in a range from about 0.1 mm to about 3 mm.

The device is configured to be releasably held in the centrifuge so that an axis normal to the surface of the solid support and preferably coming through the center of the area allocated for cell sedimentation intersects the axis of rotation and the long axis of the solid support is in the plane of rotation; or
the normal to the surface of the solid support intersects the axis of rotation and the long axis of the solid support is perpendicular to the plane of rotation.

The area of selected size into which the targeted cells deposit onto the top surface of the solid support may be functionalized with agents selected to modify the interaction between the targeted cells and the surface to assist adherence of the targeted cells to the top surface of the solid support. These agents may be selected from the group consisting of poly-L-lysine, silane coatings, gelatin, fibronectin, gold or silver coatings. For example, glass slides coated with poly-L-lysine (Merck, USA) can be used.

These agents are selected to provide a differential binding force to the targeted cells and any other interfering cells such that the targeted cells are bound to the surface while any interfering cells do not bind the surface of the top surface of the solid support.

The fluid absorbing element may be made of porous plastic, and the porous plastic may be coated with a hydrophilic coating. The fluid absorbing element may be made of non-porous absorbing materials such as paper tissue, cotton or other fibers absorbing fluids.

The porous plastic may be porous polyethylene coated with a hydrophilic coating.

The tip of the absorbing material spaced from the top surface of the solid support is placed at the distance from the surface of the solid substrate in the range of about 0.3 mm to about 3 mm.

The porosity of the fluid absorbing element and a size of the opening are selected to provide a selected flow rate.

The selected flow rate provides a fluid force applied to the target cells deposited on the surface which is below a detachment threshold level of the target cells detaching from the surface of the solid support.

The solid support may be a microscope slide.

The first removable cap may include a gasket to provide a liquid tight seal during centrifugation.

The area on the surface of the solid support outside of the area of selected size into which the targeted cells deposit onto the top surface of the solid support may be coated with a protective coating to prevent adhesion of accidentally spilled fluids to the surface of the support.

The enclosure configured to receive the fluid absorbing element includes overflow features to prevent overflow of fluid caused by insertion of the fluid absorbing element into the fluid chamber. These overflow features may include at least one opening in an upper part of the absorbing element enclosure and the openings may have any of square, circular or slot shapes.

The present disclosure provides a method, as defined in claim 18, for retention on a solid support and postprocessing of target cells present in a fluid medium with optimum retention rate for the target cells adhering to at least a portion of the surface of the solid support and preserved morphology of the target cells. The method includes subjecting the fluid medium to centrifugation to induce sedimentation of the target cells onto the surface of the solid support. Post sedimentation removal of the fluid medium is undertaken and is characterized by a controlled rate of the fluid medium removal such that the target cells sedimented on the surface are not detached from the surface by fluid flow in a vicinity of the sedimented cells. The volume of residual fluid is controlled at all stages of cell retention and post processing. The sedimented target cells are characterized in that the morphology of the target cells is substantially unchanged from their morphology when free floating in the fluid medium.

The flow rate is controlled by the porosity of the material the absorbing element is made of, the area of the opening in the enclosure of the absorbing element and cross-section of the absorbing element.

At least a portion of the surface of the solid support onto which the target cells sediment is of a selected size which is defined by the size of an opening in the fluid chamber in which the fluid containing target cells is placed for centrifugation. The area of selected size may be functionalized with agents selected to modify the interaction between the target cells and the surface to assist adherence of the targeted cells to the top surface of the solid support.

These agents may be selected from the group consisting of poly-L-lysine, silane coatings, gelatin, fibronectin, gold or silver coatings. These agents are selected to provide a differential binding force to the targeted cells and other cells such that the targeted cells are more readily bound to the surface while binding of other cells to the top surface of the solid support is less efficient.

The parameters of the absorbing element and the absorbing element housing are selected so that the shear force associated with the fluid removal rate is low enough so that the detachment of the target cells is minimal but the same shear force is sufficient for removal of other cells that are not of interest, thereby leading to enrichment of the target cells of interest.

A further understanding of the functional and advantageous aspects of the present disclosure can be realized by reference to the following detailed description and drawings.

### BRIEF DESCRIPTION OF THE DRAWINGS

Embodiments of the cell harvest device disclosed herein will now be described, by way of example only, with reference to the drawings, in which:
**FIG. 1** is a flow diagram of the cell retention method disclosed herein.
**FIG. 2(a)** is a perspective view of the assembled cell retention device **40** for cell sedimentation, post centrifugation with reduced cell losses (increased retention rate) and improved morphology of retained cells.
**FIG. 2(b)** shows an assembled cell retention device configured for centrifugation ready to be positioned in a centrifuge (not shown).
**FIG. 2(c)** shows an assembled cell retention device positioned in a centrifuge (not shown) so that the normal **41** to the surface of the solid support **42** intersects the axis of rotation and the long axis of the solid support **42** is in the plane of rotation.
**FIG. 2(d)**shows an assembled cell retention device positioned in a centrifuge (not shown) so that the normal **41** to the surface of the solid support **42** intersects the axis or rotation and the long axis of the solid support **42** is perpendicular to the plane of rotation.
**FIG. 3** is an exploded view of the cell retention device for cell sedimentation with reduced cell losses (increased retention rate) and improved morphology of retained cells.
**FIG. 4(a)** is a perspective view of an absorbing element **46** in a plastic enclosure **48** forming part of the cell retention device.
**FIG. 4(b)** is a front view of the absorbing element **46** in the plastic enclosure **48** of **FIG. 4(a)****.**
**FIG. 5(a)** is a perspective view showing the tip of the plastic enclosure **48** of the absorbing element **46** with rectangular opening **8.**
**FIG. 5(b)** is a perspective view showing the tip of the plastic enclosure **48** of the absorbing element **46** with a circular cross-section opening **8a**.
**FIGS. 6(a) to 6(e)** show various implementations of controlling the wicking rate during the process of fluid removal after completion of cell sedimentation according to the proposed method, in which:

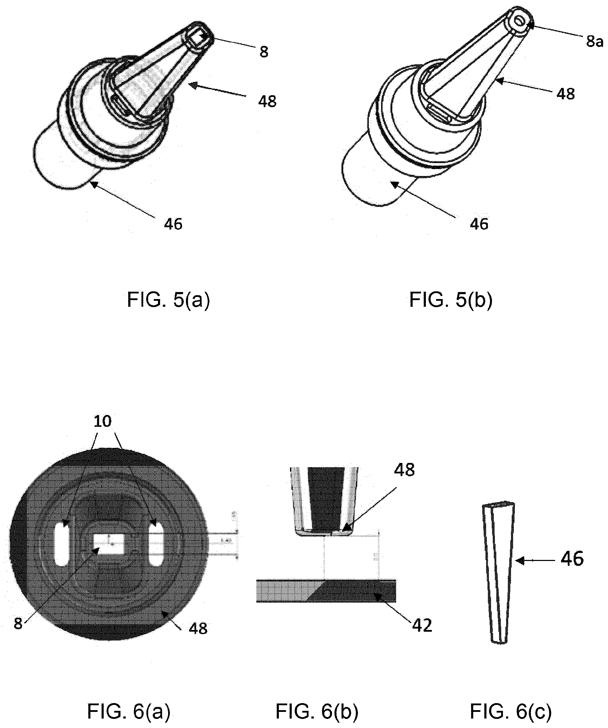

   **FIG. 6(a)** shows controlling of fluid removal rate by selection of the cross-sectional area of the opening **8** in the plastic enclosure **48;**

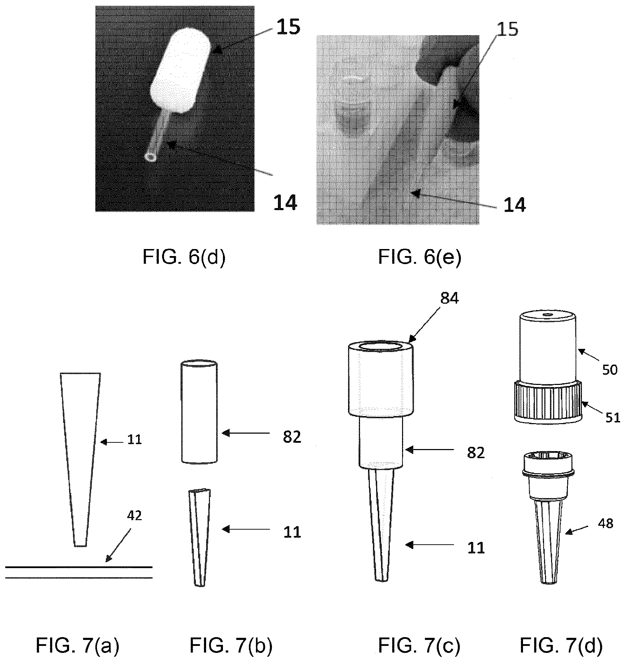

   **FIG. 6(b)** shows how this result is achieved by selection of the distance between the plastic enclosure **48** containing and absorbing element **46** (not shown in **FIG. 6**); the result mentioned above can also be achieved by a combination of cross-sectional area and the distance mentioned above;
   **FIG. 6(c)** shows control of the fluid flow rate by selection of the geometry of the absorbing element **46;**
   **FIG. 6(d)** shows control of the fluid flow rate by an additional capillary tube 14 attached to the porous material **15** and selection of capillary length and inner diameter; and
   **FIG. 6(e)** shows a capillary **14** with continuously increasing cross-sectional area filled with porous material.
**FIGS. 7(a)** to **7(e)** show progressively more components forming absorbing element which is a part of the present device including a flat tip **11,** cylindrical rod **82** and hollow cylindrical rod **84** allowing increase of the wicking capacity of the absorbing element, wherein:
   **FIG. 7(a)** shows a tip of the absorbing element **46** made as a flat porous component **11** which is placed in proximity to the solid support **42;**
   **FIG. 7(b)** shows an exploded view of the absorbing element **46** composed of two porous components **11** and **82;**
   **FIG. 7(c)** shows an assembled multicomponent absorbing element **46** composed of three parts: flat porous component **11,** cylindrical porous part **82** and hollow cylindrical porous part **84;**
   **FIG. 7(d)** shows the plastic enclosure for the absorbing element **46** (not shown in this drawing) containing a wicking cavity **48** and a cap **50** with riffled surface **51;** and

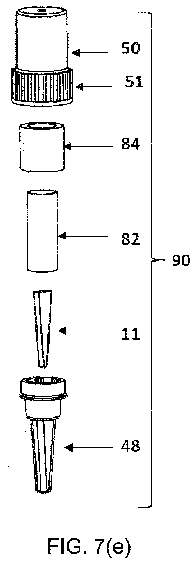

   **FIG. 7(e)** shows an exploded view of the encapsulated multicomponent absorbing element **90.**

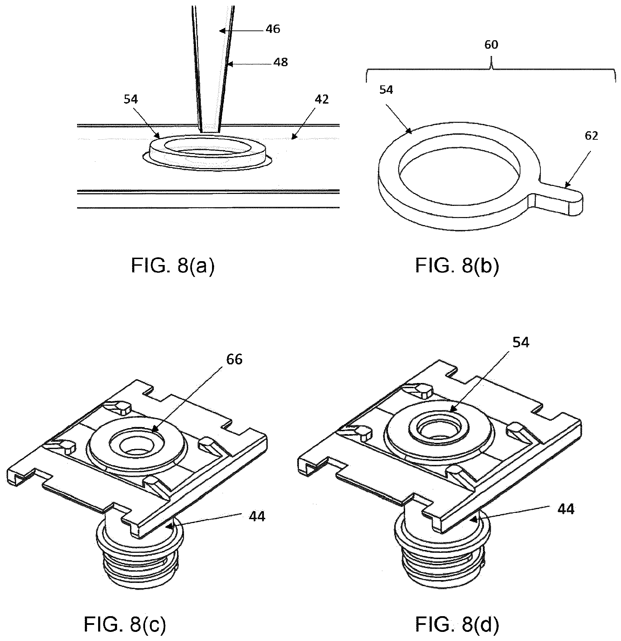

**FIG. 8(a)** shows the tip of the absorbing element **46** in the enclosure **48** and a sealing gasket **54** attached to the surface of the substrate **42** which creates a cavity for the reagents.
**FIG. 8(b)** shows another embodiment of a gasket **60 in which a** protruding element **62 is added to gasket 54.**

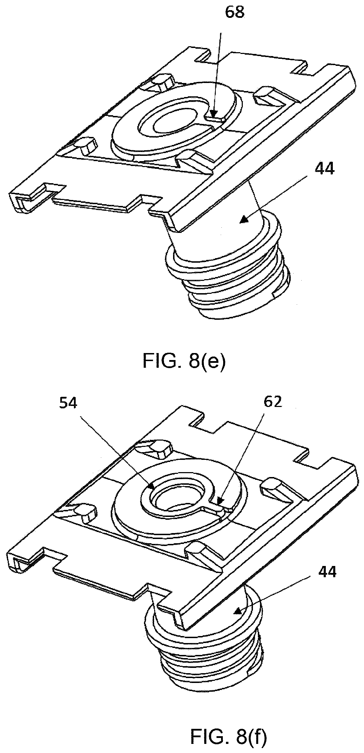

**FIGS. 8(c)** to **8(f)** are perspective views showing the bottom of fluid chamber **44** and two embodiments of groves in the bottom of fluid chamber **44** for accepting sealing gaskets, one embodiment shown in **FIGS. 8(c)** and **8(d)** and the other embodiment shown in **FIGS. 8(e)** and **8(f)****.**
**FIG.** 9 shows is a view of the absorbing element housing **48** showing overflow protection features **10.**
**FIG. 10(a)** shows a first embodiment of the absorbent material housing **48** having flow accelerating features **17** for increase of the wicking rate without impact onto cell loss, which are circular holes.
**FIG. 10(b)** shows a second embodiment of the absorbent material housing **48** having flow accelerating features **17a,** which are elongated slots.

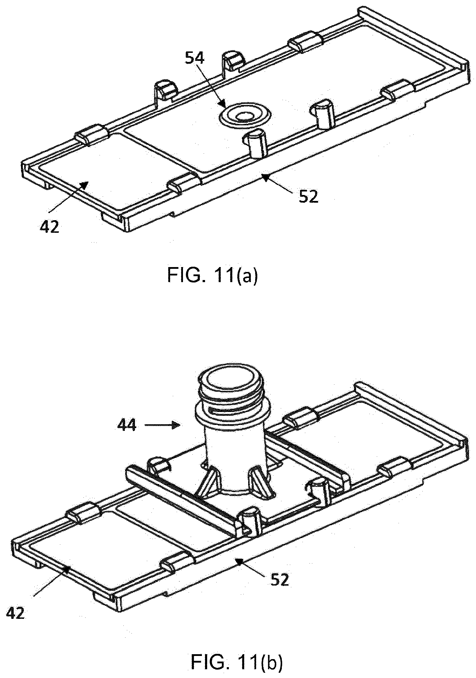

**FIG. 11(a)** shows a perspective view of the solid support **42** in a form of a glass slide with region of the surface being functionalized with the fluid chamber **44** detached with gasket **54** surrounding the functionalized region.
**FIG. 11(b)** shows the re-attached fluid chamber **44.**

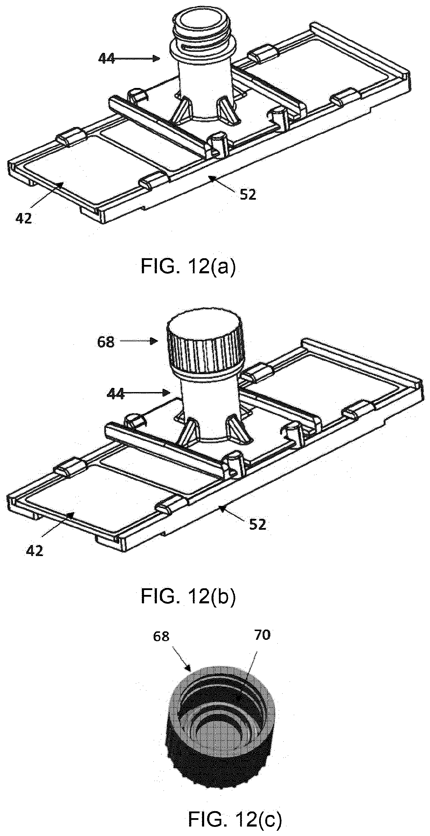

**FIG. 12(a)** is a perspective view showing the cell retention device partially disassembled at the stage of fluid introduction into fluid chamber **44.**
**FIG. 12(b)** is a perspective view showing the cell retention device assembled with a screw cap **68** after introduction of the fluid sample containing the cells to be harvested.
**FIG. 12(c)** is a perspective view showing the underside view of the screw cap **68** with an O-ring **70** used for leak elimination during centrifugation.

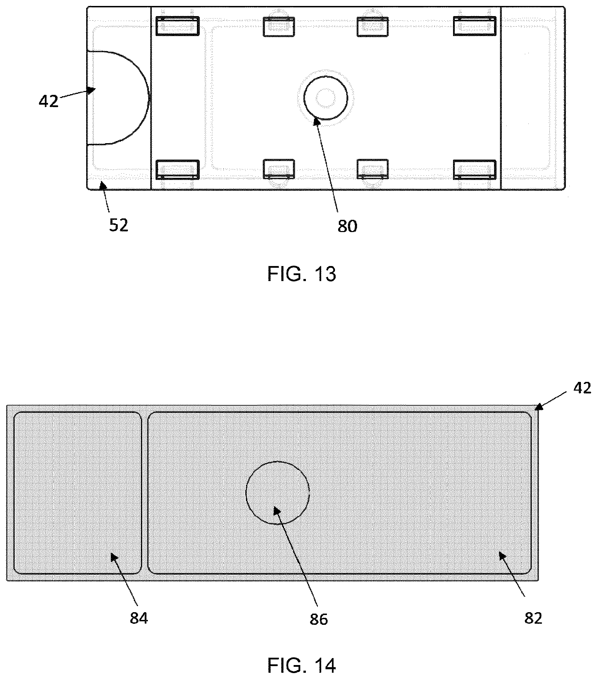

**FIG. 13** shows the base **52** of the cell retention device with a hole **80** for fluid and cell visualization without detachment of the fluid chamber **44.**
**FIG. 14** shows the solid support **42** of the cell retention device with a section **86** of the surface functionalized for capturing cells and Teflon protected layer **82** around the functionalized area **86** and area **84** used for labeling.

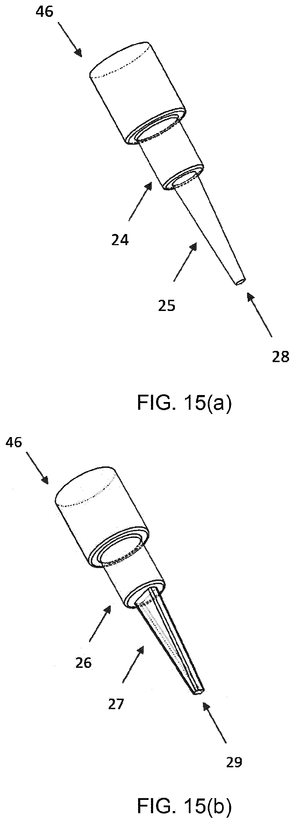

**FIG. 15(a)** shows a molded single-component absorbing element **46** with a conical tip **25** with circular cross-section **28.**
**FIG. 15(b)** shows the molded single-component absorbing element **46** with a and a flat tip with a triangular shape **27** and rectangular cross-section **29.**

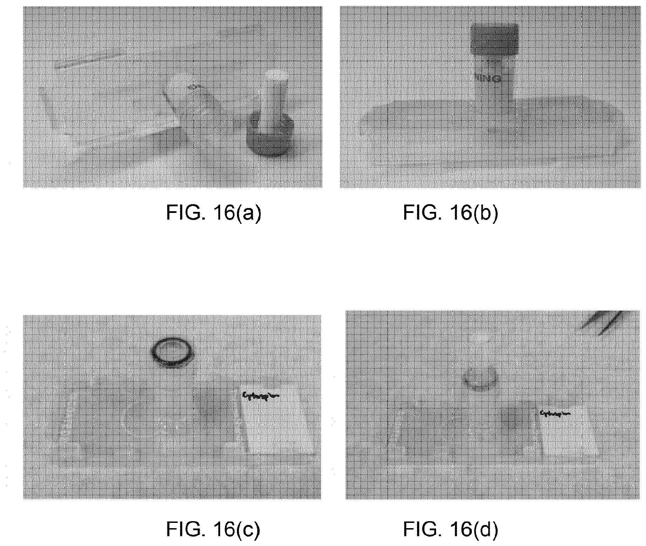

**FIGS. 16(a)** to **16(d)** are photographs showing example embodiments of the present a cell recovery device **40,** with a glued in fluid chamber **44,** in which:
   **FIGS. 16(a)** and **16(b)** show another embodiment of the proposed device with the absorbing element detached from the assembly **(****FIG 16 (a)****)** and assembled device with the absorbing element in contact with the fluid **(****FIG 16 (b)****);** and
   **FIGS. 16(c)** and **16(d)** show another embodiment of the proposed device in which the fluid chamber **44** is glued directly to the surface of slide 42. **FIG 16 (c)** shows the cap **68** attached to the fluid chamber **44** prior to centrifugation step of the process. **FIG 16 (d)** shows the encapsulated absorbing element **46** made of absorbing paper tissue inserted into the fluid chamber **44** for fluid removal after completion of the centrifugation stage.

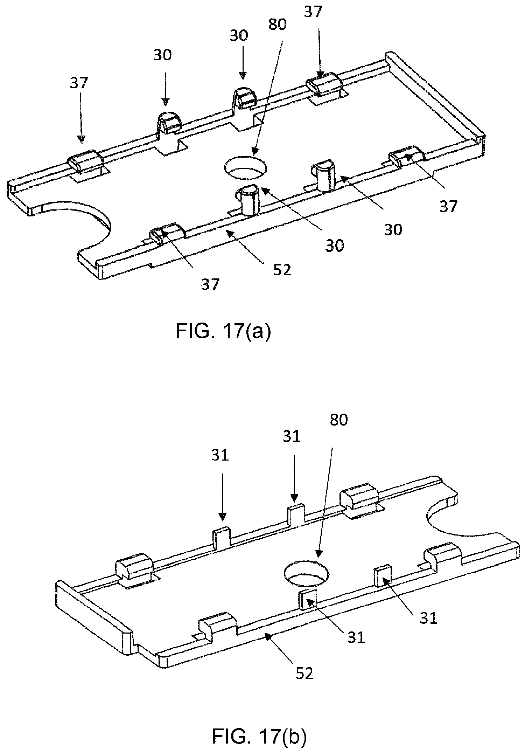

**FIGS. 17(a)** to **17(f)** are perspective views showing in various examples of different methods of retaining parts of the assembly of the cell retention device.

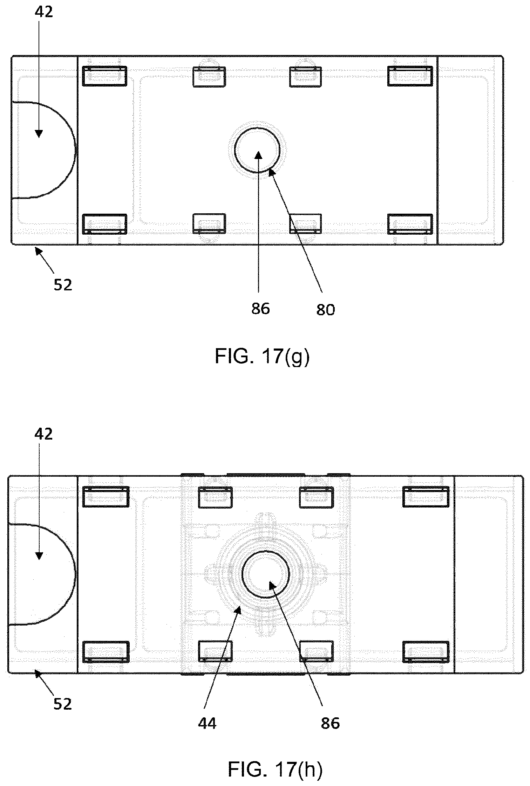

**FIG. 17(g)** is a bottom view showing the alignment of a slide **42** with functionalized surface **86** region in the assembled device with a bottom opening 80 in base **52.**
**FIG. 17(h)** is a bottom view of base 52 showing the alignment of a slide **42** with the functionalized surface region **86** in the assembled cell retention device with a bottom opening in base **52** a fluid chamber **44.**

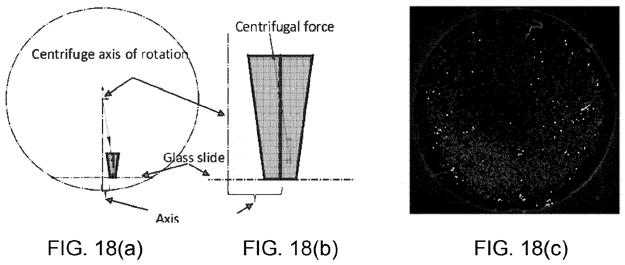

**FIGS. 18(a)** to **18(f)** shows a series of panels showing the importance of proper alignment of the cell retention device axis relative to the axis of rotation in a centrifuge in which:
   **FIG. 18(a)** shows a schematic representation of the fluid chamber **44** with the cells in a centrifuge with the axis of the device shifted from the axis of the centrifuge;
   **FIG. 18(b)** shows force distribution during centrifugation.
   **FIG. 18(c)** shows the resulting inhomogeneous distribution of the sedimented cells;

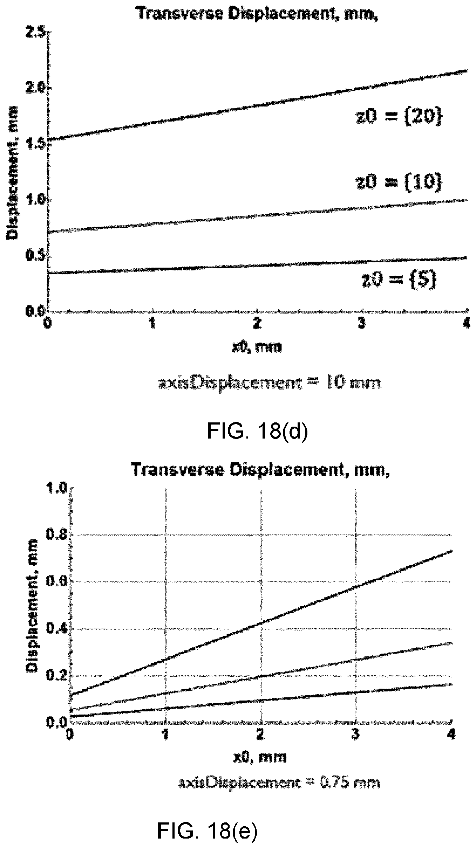

   **FIG. 18(d)** shows plots of calculated displacement of the cells in the direction perpendicular to the axis of rotation from the original position as a function of the initial distance between the cell and the axis of the deice for different displacement of device axis relative to the axis of rotation in a centrifuge for 10 mm;
   **FIG. 18(e)** shows the same for a displacement of 0.75 mm and panel; and

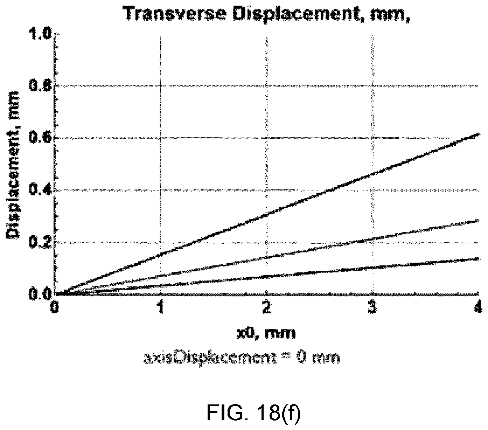

   **FIG. 18(f)** shows the same for a displacement of 0 mm.

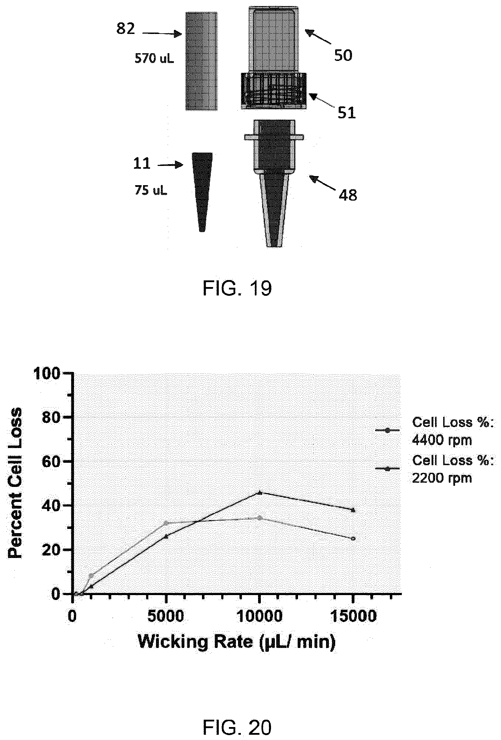

**FIG. 19** shows an elevation view of an example of the absorbing element **46** composed of two porous components **11** and **82** and a plastic enclosure for this absorbing element.
**FIG. 20** is a plot of percent cell loss versus wicking rate for two different centrifugation speeds 4400 rpm (circles) and 2200 rpm (triangles).

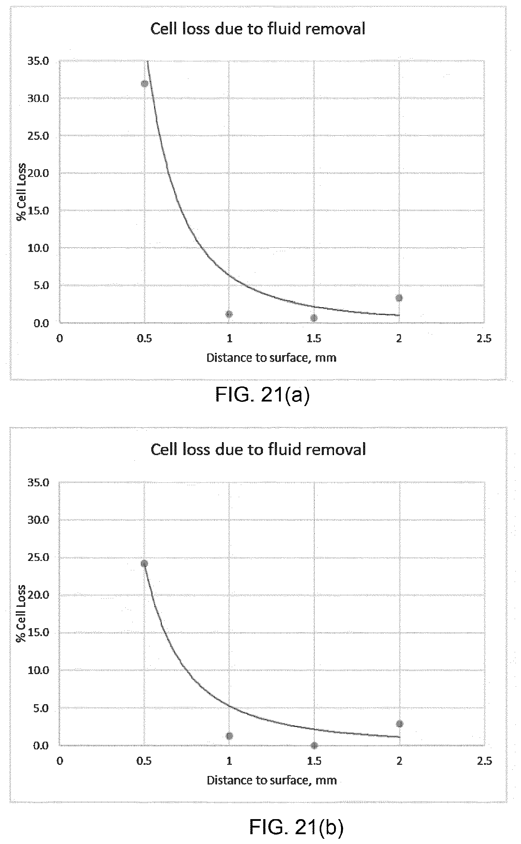

**FIG. 21(a)** shows percent cell loss as a function of the distance between the surface of the solid support **42** and the absorbing element **46,** forming part of the present cell retention system, without addition of KCI.
**FIG. 21(b)** shows percent cell loss as a function of the distance between the surface of the solid support **42** and the absorbing element **46,** forming part of the present cell retention system and with KCI added to the solution.

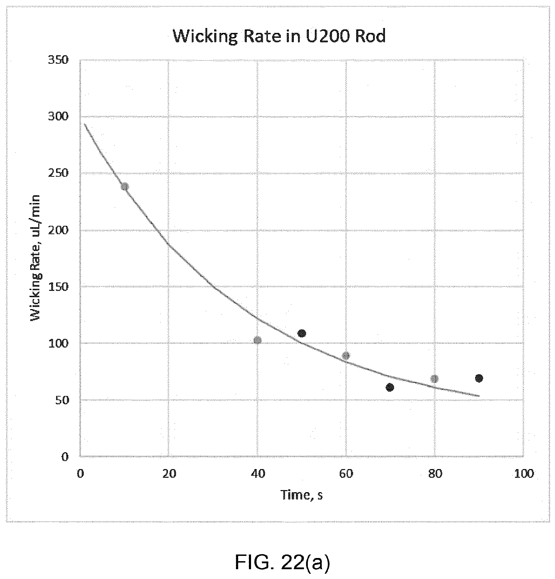

**FIG. 22(a)** shows the wicking rate as a function of time for polypropylene porous material U200 (Pore Technology Inc.), with the distance between the surface of the solid support **42** and the tip of the absorbing element **46** being about 2 mm.

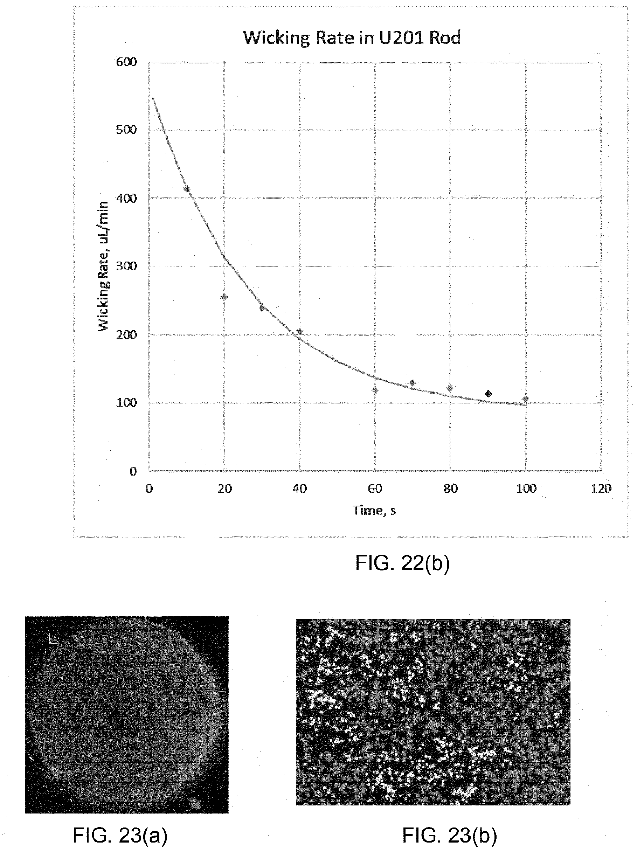

**FIG. 22(b)** shows the wicking rate as a function of time for polypropylene porous material U201 (Pore Technology Inc) with the distance between the surface of the solid support **42** and the tip of the absorbing element **46** being about 2 mm.
**FIG. 23(a)** is a microphotograph of DAPI stained cells spiked into fluid chamber **44** and sedimented by centrifugation and shows cells retained on the slide surface after centrifugation with consecutive fluid removal according to the present method.
**FIG. 23(b)** is a magnified image (X100) of the microphotograph of **FIG. 23(a)****.**

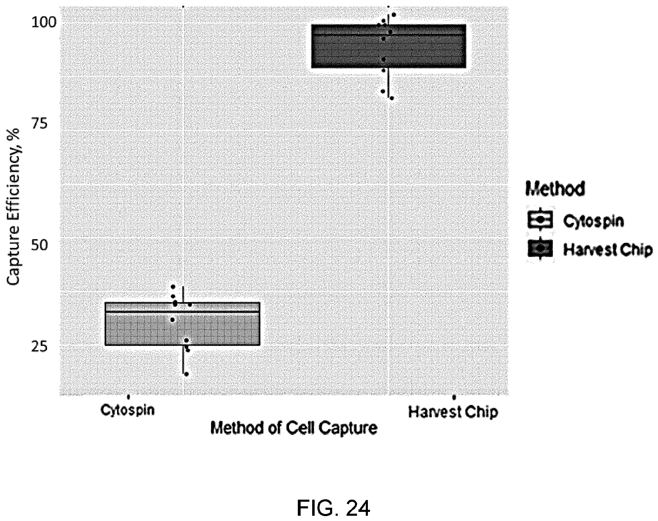

**FIG. 24** shows percent cell capture efficiency for two methods of cell capture: in Cytospin^{®} shown in data set 1 and using the present cell retention method and device shown in data set 2.

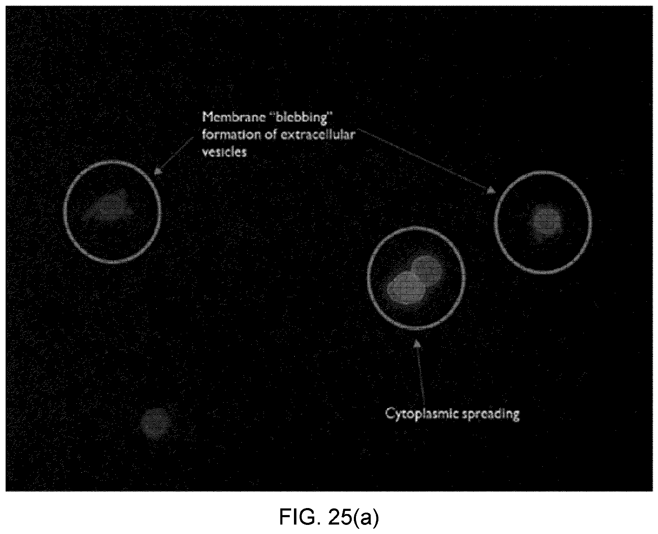

**FIG. 25(a)** shows microphotographs of cells captured using Cytospin^{®}.

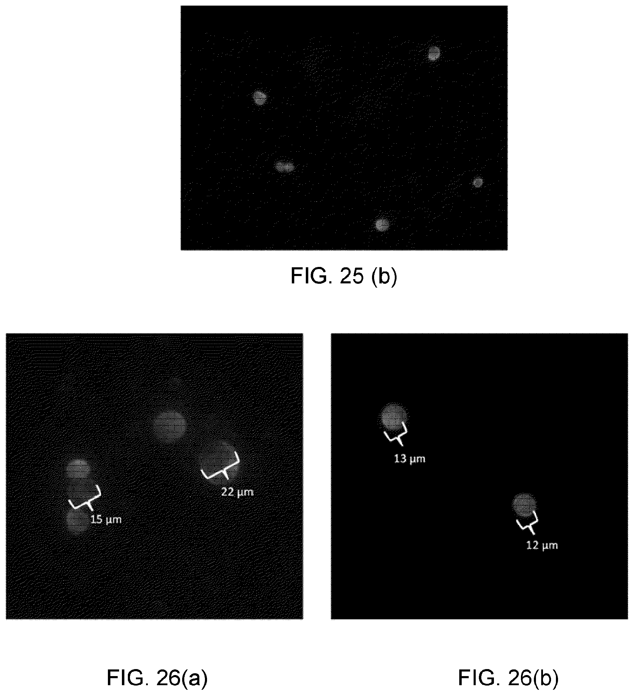

**FIG. 25(b)** shows microphotographs of cells captured using thecell recovery device **40** of the present cell retention device, which clearly shows the present method preserves the morphology of the harvested cells.
**FIG. 26(a)** shows microphotographs showing the morphology of SK-BR-3 cells which were fixed with lack of fluid around them (referred to as dry fix), with the images being obtained using 400x magnification and CK-FITC conjugate stain.
**FIG. 26(b)** shows microphotographs showing the morphology of SK-BR-3 cells which were fixed in the presence of controlled volume of fluid (wet fix), with the images being obtained using 400x magnification and CK-FITC conjugate stain.

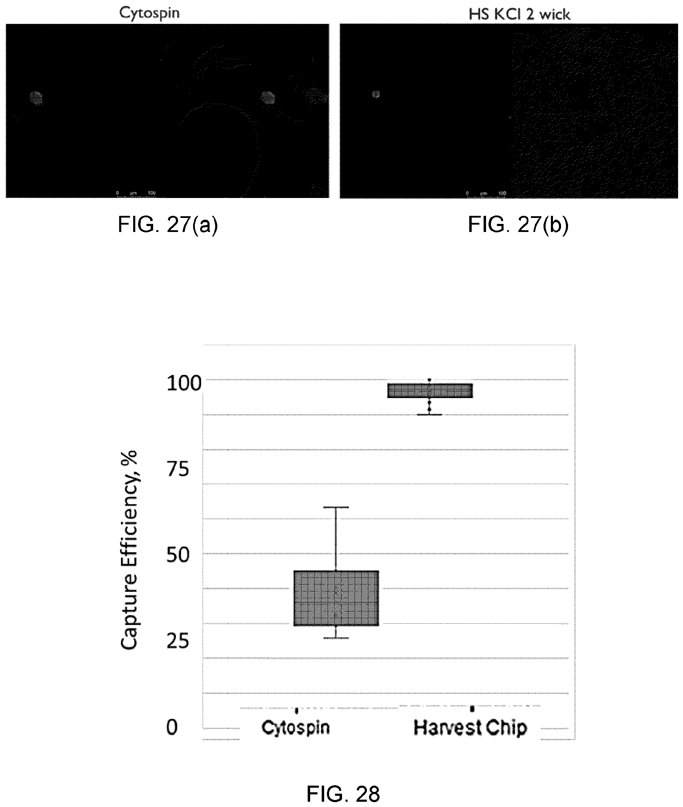

**FIG. 27(a)** shows a microphotograph of the circulating tumor cells (CT) retained with Cytospin^{®} using Streck Biomedica mBC patient samples.
**FIG. 27(b)** shows a microphotograph of the circulating tumor cells (CT) retained with the present cell retention device using Streck Biomedica mBC patient samples, which shows the morphology of the sedimented cells is noticeably better when the method disclosed herein is used.
**FIG. 28** shows a histogram of the capture efficiency of EDTA HNV blood spiked with pre-labelled SK-BR-3 cells separated via Parsortix^{®}, pooled, captured, fixed and then stained on either a cell recovery device **40** of the present cell retention device or Cytoslide^{™}. The experiments were performed by multiple trained users across multiple instruments/ days. Impact of the pipetting variability is reduced (compare STD on Cytospin^{®} and a cell recovery device **40**).

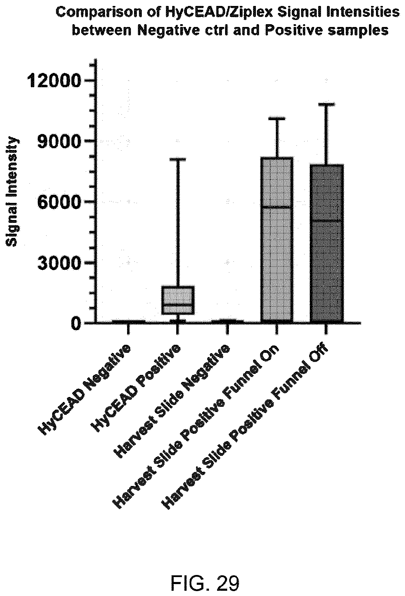

**FIG. 29** show histograms plotting HyCEAD^{™}/ Ziplex^{®} signal intensities for the negative control and positive control for the HyCEAD^{™} process used for the consecutive molecular analysis of retained cells, negative control for the complete process of sample retention and consecutive molecular analysis and two modes of cell retention with the cell containing samples with consecutive molecular analysis.

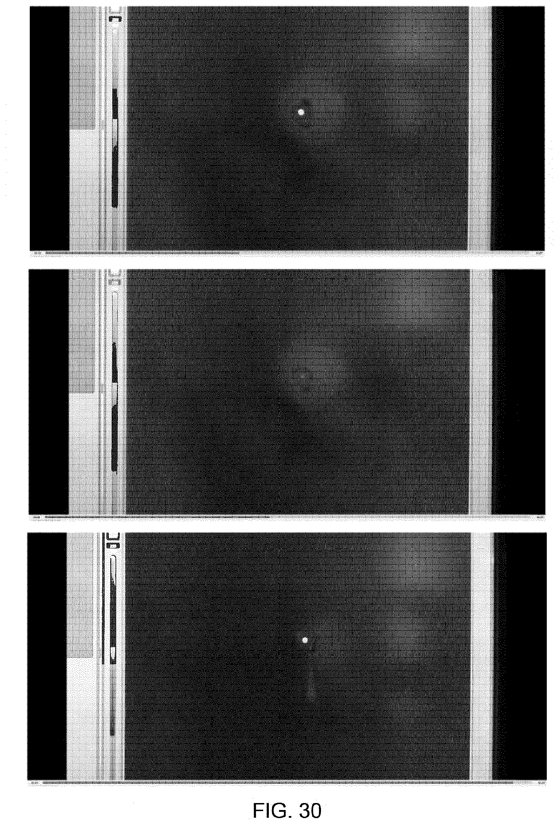

**FIG. 30** shows picking of SK-BR-3 cells deposited onto the solid support **42** with a controlled volume of the residual fluid according to a proposed method. The top panel shows a retained cell with a capillary of a cell-picking tool placed above this cell; the middle panel shows the cell being removed from its initial location by the cell-picking tool, and the bottom panel shows the cell dispensed (re-positioned) by the cell-picking tool.

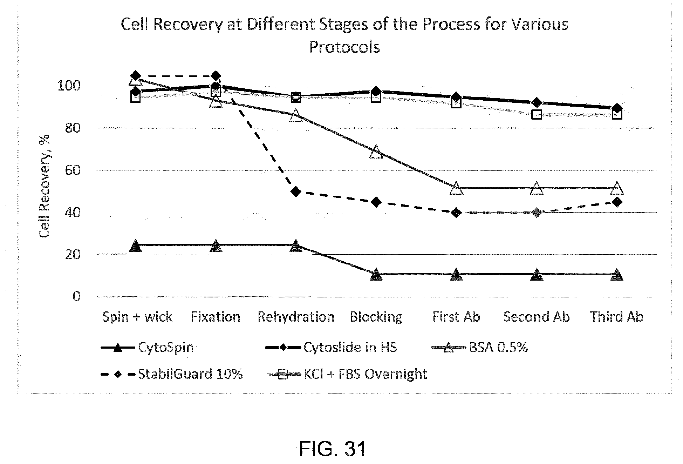

**FIG. 31** shows % cell recovery at different stages of the process with various protocols as noted in **FIG. 31**.

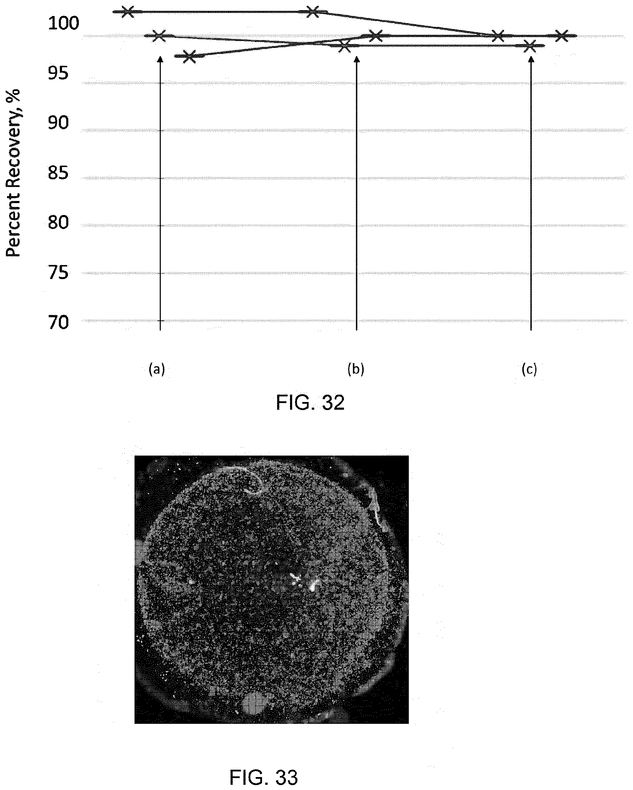

**FIG. 32** shows percent of cell recovery for multiple consecutive wicking steps (a, b, c) in which the first wicking (a) is done after cell sedimentation, the second wicking (b) is done after resuspension and the third wicking (c) is done after staining, and wherein the test was repeated three times demonstrating that multiple wicking events do not lead to cell losses if the wicking rate is properly selected according to the present method.
**FIG.** 33 is a microphotograph of white blood cells sedimented using the present method disclosed herein and stained with DAPI in which the photograph is taken with the fluid chamber **44** still attached to the solid support demonstrating that the cells can be stained with the fluid chamber **44** attached.

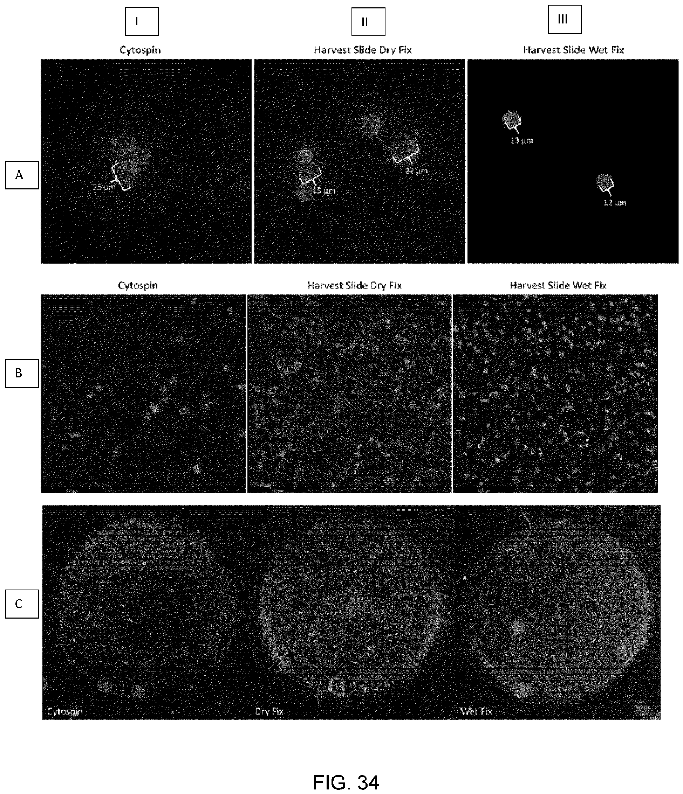

**FIG. 34** shows microphotographs (rows A, B) and a photograph (row C) for the cells sedimented using Cytospin^{®} (column I) on the solid support of a cell recovery device forming part of the present cell harvesting device (columns I and II) without controlling residual fluid (column II) and with control of residual fluid volume (column III) according to the method disclosed herein. The cell recovery device spiked HNV cell data - a cell recovery device with wet fixation method on live EDTA tube cells after staining. Cytospin^{®} presented the worst morphology (column I, rows A and B) with cytoplasmic spreading and reduced fluorescence intensity as well as increased background. Least amount of morphological alteration was seen in wet fixed a cell recovery device **40** condition (column III). Similar results seen concerning CD45 staining showing reduced cell density in Cytospin^{®} is also of concern.

### DETAILED DESCRIPTION

Various embodiments and aspects of the disclosure will be described with reference to details discussed below. The following description and drawings are illustrative of the disclosure and are not to be construed as limiting the disclosure. Numerous specific details are described to provide a thorough understanding of various embodiments of the present disclosure. However, in certain instances, well-known or conventional details are not described in order to provide a concise discussion of embodiments of the present disclosure.

As used herein, the terms, "comprises" and "comprising" are to be construed as being inclusive and open ended, and not exclusive. Specifically, when used in the specification and claims, the terms, "comprises" and "comprising" and variations thereof mean the specified features, steps or components are included. These terms are not to be interpreted to exclude the presence of other features, steps or components.

As used herein, the term "exemplary" means "serving as an example, instance, or illustration," and should not be construed as preferred or advantageous over other configurations disclosed herein.

As used herein, the terms "about" and "approximately" are meant to cover variations that may exist in the upper and lower limits of the ranges of values, such as variations in properties, parameters, and dimensions. In one non-limiting example, the terms "about" and "approximately" mean plus or minus 10 percent or less.

Unless defined otherwise, all technical and scientific terms used herein are intended to have the same meaning as commonly understood to one of ordinary skill in the art.

As used herein, the phrase "target cells" means the cells of interest being isolated by the present device. In some cases, all the cells present in the sample may be of interest if selectivity in cell isolation is not required for that particular intended use or for the following post-processing method and thus all cells present in the sample would be considered "target cells". While other cells may be present so that there is a mixture different types of cells, it is only the "target cells" that are being isolated or predominantly isolated.

As used herein, the phrase "accelerating features" refers to structural elements designed to increase the rate of fluid removal from the fluid chamber **44** after completion of centrifugation without loss of the cells deposited on the surface of solid support **42.** This allows for a reduction of the time required for sample processing. These accelerating features are located on the enclosure **48** providing an additional fluidic connection between the fluid present in the fluid chamber **44** and the absorbing element **46.** The accelerating features may be of a round shape, or have a shape of a slot. Other shapes are possible as well. The accelerating features are configured so that the open are of the features is sufficient to increase the flow rate to the required value and the accelerating features are located of the enclosure **48** at such a distance from the tip of the enclosure **48** that the additional shear stress applied to the cells on the solid support **42** due to the increased flow rate doe not cause additional cell losses.

As used herein, the phrase "overflow protection features" refers to structural elements designed to prevent overflow of fluid from the fluid chamber **44** when the absorbing element **46** placed in the enclosure **48** is inserted into fluid chamber **44.** These overflow protection features are located on the enclosure **48** providing an additional fluidic connection between the fluid present in the fluid chamber **44** in case when the volume of the sample is excessive. These overflow protection features protect against creation of turbulent flow in the fluid chamber **44** which might cause cell detachment from the surface of the solid support **42.**

The present disclosure describes a device comprising a fluid chamber assembled with a microscope slide (or other substrate) that has been sealed liquid-tightly using an elastomeric gasket. The slide surface has been functionalized with a coating allowing capturing and retention of cells and is held in a polypropylene holder that clips onto the fluid chamber thus physically securing and positioning the chamber relative to the slide. Apart from the assembly containing the microscope slide, it also features a porous absorbing element that is contained within a polypropylene shell that improves usability while providing a means by which to control the flow rate of liquid into the porous absorbing element. The absorbing element in the polypropylene shell is introduced into the assembly after completion of the centrifugation stage and can be replaced with the identical absorbing element and shell if the process of cell post processing requires multiple steps of fluid introduction and removal.

The device is intended to serve as a cytocentrifugation device where cells of interest that are suspended in a surrounding medium are of a higher density than the surrounding medium, thus when centrifugal force is applied to the device containing the cell suspension, the denser cells of interest are preferentially sedimented according to the direction of the centrifugal force. Cytocentrifugation devices can be largely categorized into two groups based on their method of fluid removal. In a "Simultaneous Fluid Removal" system, an absorbent medium is situated between the fluidic chamber and slide surface, allowing for the removal of liquid into the adjacent absorbent medium simultaneously as the centrifugal force is applied. Theoretically, the denser cells of interest must have sedimented to the slide surface prior to the removal of liquid as doing so otherwise would result in cells still suspended in medium being drawn into the adjacent absorbent element and being lost. The other approach to cytocentrifugation features a liquid-tight seal between the fluidic chamber and slide surface that does not allow for the removal of liquid during centrifugation. In fluid-retention cytocentrifugation, the cells of interest are sedimented while surrounded in their supportive liquid medium and the removal of fluid is performed after centrifugation. The comparatively denser cells of interest in liquid medium are still preferentially sedimented to the slide surface.

Fluid-retention cytocentrifugation presents a number of benefits but also poses some challenges from the standpoint of sample manipulation. As the cells are sedimented in a liquid medium that provides relative buoyancy (i.e., resistance to sedimentation) as opposed to a complete lack of buoyancy when centrifuged in air, a higher amount of centrifugal force is required for the denser cells to overcome the fluidic resistance. The presence of cells in liquid medium presents an advantage, as the cells being sedimented experience less force pressing them against the slide, aiding in the preservation of delicate morphological features. The lack of simultaneous fluid removal also presents the benefit of effectively removing all avenues by which cells of interest can be lost during the centrifugation process. By principle of being liquid-tight, the fluid which contains cells of interest are restricted to remain within the chamber and contact the slide surface - as such, cells suspended in the liquid medium are also restricted to remaining within the chamber and settling to the slide surface.

A limitation presented by the fluid-retention method of cytocentrifugation is that the processes of fluid removal and cell sedimentation are separated. As such, a separate method of fluid removal is necessary. Considering that the cells do not experience as much centrifugal force owing to the buoyancy of the surrounding medium, it is possible that the cell contact area with the slide is not as large, thus adversely affecting the adhesion of cells to the slide surface. This requires that the process of liquid removal be performed as gently and with as little turbulence as possible. The device and its method of use provides a method of fluid removal from the sample chamber in a controlled manner that minimizes the detachment of cells from the slide surface, thus maximizing the yield of settled cells of interest.

Upon the removal of liquid, the cells are sedimented within a designated area of the slide within the sample chamber. To aid in further downstream processing wherein multiple reagents may be used to incubate or wash the cells, or imaging of sedimented cells is required with the following postprocessing so that it is beneficial that the sample chamber is removable. The present device includes a polymer base with protruding hooks that clip into corresponding positions on the sample chamber sitting atop the slide. The clips are designed for ease of assembly but also provide a convenient means by which the end-user can remove the chamber without the use of specialized tools or methods.

To facilitate the manipulation of reagents in downstream processes such as immunofluorescence staining and fluorescence in-situ hybridization where it is optimal that fluid volumes are minimized to reduce assay costs, the device can include hydrophobic printing on surfaces other than the exposed bioadhesive area. The hydrophobicity of the printed surface serves as a convenient reagent well that can hold small volumes of liquid (the capacity of the well is 10 to 200 µL) but also by the means of retaining the liquid in a controlled area, the cells of interest are also limited to a designated area, allowing for the strict localization of the sample and minimizing loss due to downstream processes.

To maximize the ability of the cells of interest to adhere to the slide surface and resist detachment, the slide surface was functionalized with a bioadhesive coating to modify the surface interactions of the sedimented cell with the slide. The design of the device wherein a slide is held in a polymer base that clips together with a chamber that is sealed onto the slide via a gasket allows the device to be compatible with virtually any microscope slide that fits within the base. Thus, a wide range of functionalized slides were tested in the device and evaluated for cell adhesion performance. As the slide is easily detachable from the rest of the device components it allows the device to be compatible with virtually any microscope including both upright and inverted microscopes. Also, the slide with the sedimented cells is compatible with other types of standard equipment such as, for example, slide staining devices. Cultured SK-BR-3 cells pre-labelled with CellTracker^{™} Green were spiked into 1X PBS to create a cell suspension and added to the sample chamber. Upon centrifugation, the supernatant was removed, and the cells fixed by incubation in ice-cold Methanol for 5 minutes followed by detachment of the sample chamber. The resultant slide containing sedimented cells was washed by running 1X PBS over the sample surface 3 times using a micropipette to induce cell detachment. The subsequent slide surfaces were imaged under FITC and the cell density evaluated. As a result, Poly-L-Lysine showed the highest density of cells. Thus, this surface modification was chosen as the primary slide surface for the present device. Other types of coatings can be used for surface functionalization instead of Poly-L-Lysine such as proteins, silanes, organic polymers, inorganic metals. For example, other forms of poly-lysine, silane coatings, gelatin, fibronectin, gold or silver coatings and other coatings enhancing cell adhesion to a solid support. Other methods of enhancement of cell adhesion to the surface of the solid support can be used such as surface structure modification, etching, plasma treatment.

Different materials can be used as a solid support such as glass, different types of plastic or quartz.

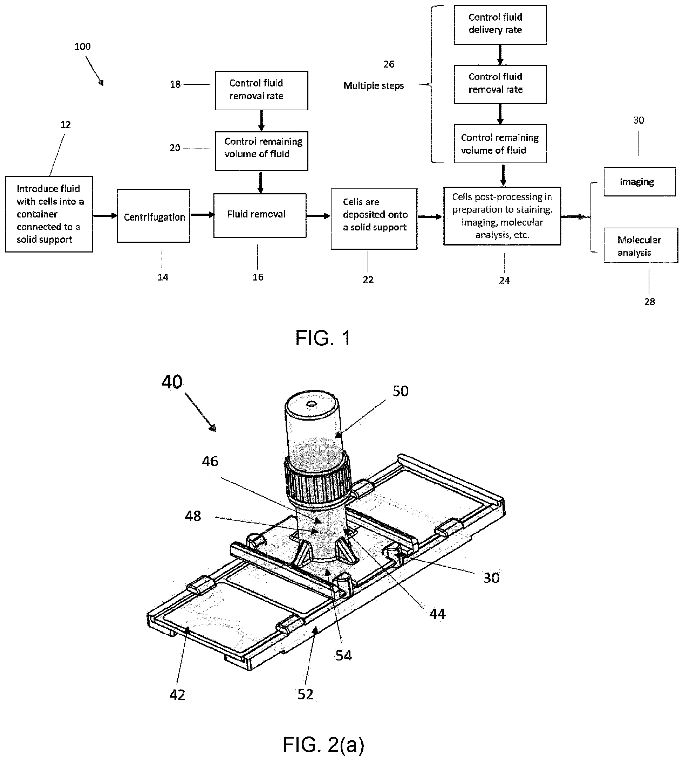

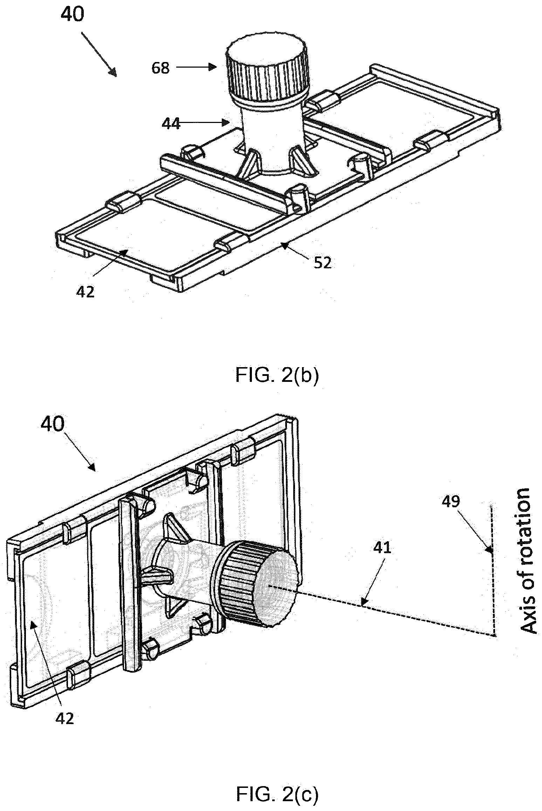

Referring to **FIGS. 1** and **2(a), FIG. 1** shows a flow diagram at **100** of the cell retention method using the cell harvesting device disclosed herein. In step **12** a fluid sample with the cells being isolated is introduced into a fluid chamber **44 (****FIG. 2(b)****)** which is coupled to a solid support **42** both of which form part of the device described below. In step **14** the fluid sample is then subjected to centrifugation which forces the cells to move towards the solid support **42** which induces sedimentation of the cells and promotes cell adhesion to the solid support. After centrifugation, after which in step **16** the fluid is removed under controlled conditions by carefully controlling the fluid removal rate in step **18** and actively controlling the remaining fluid in step **20.** Carefully controlling the fluid is advantageous in that it prevents cell detachment from the solid support due to excessive forces which may appear when the flow rate is high, and the stress level exceeds adhesion forces keeping the cells of the surface of the solid support during centrifugation or after centrifugation is completed. Also, removal of the fluid after completion of the centrifugation step eliminates removal of the cells which still may be present in the solution (not bound to the surface) if centrifugation is not finished. In addition, the proposed method requires control of the volume of the fluid remaining in the fluid chamber after completion of the fluid removal step. This is required for preserving cell morphology as the cells remain wetted at all steps of the process. The volume of the remaining fluid depends on the fluid parameters (surface tension and contact angle) as well as the inner diameter of the fluid chamber **44** and the distance between the solid support **42** and the tip of the absorbing element **46.** The volume basically is controlled by a proper selection of the above-mentioned distance between the tip of the absorbing element **46.**

If multiple steps are required for cell post processing (for, example staining, washing, etc. as shown in step **24**) addition of reagents is conducted with a controlled fluid delivery rate. This allows to prevent detachment and removal of the cells adhered to the solid support in the preceding steps of the method. The following steps of fluid removal are conducted with a controlled fluid removal rate and control of the remaining volume of fluid as described above, with the above being illustrated in step **26.**

Following the preparation step **24,** the prepared cells are then subject to the desired analysis, whether it be molecular analysis in step **28** or imaging in step **30.**

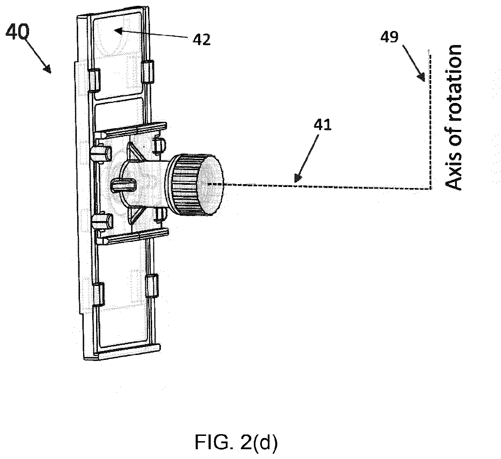

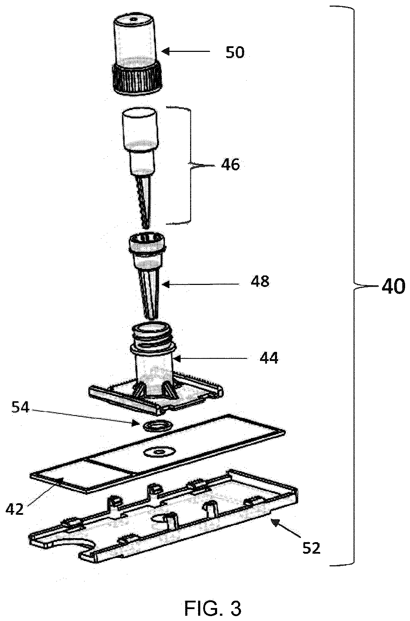

Referring to **FIGS. 2** and **3** the assembled cell retention device shown generally at **40** for cell sedimentation with reduced cell losses (increased retention rate) and improved morphology of retained cells and includes substrate **42,** a fluid chamber **44,** and includes either a wicking cap **50** shown in **FIG. 2(a)** or a protection cap **68** shown in **FIG. 2(b)** and absorbing element **46** extending from the cap **50** down into an enclosure **48** and being in flow communication with the cell containing liquid which is placed in the fluid chamber **44** and wherein the absorbing element extends for the absorbing element rod **46,** a wicking cap **50,** a base **52** and a fluid chamber gasket **54.** The absorbing element **46** can be made as a single piece element as shown further in **FIG. 15** or may consist of multiple components as shown in **FIG. 7(b), FIG. 7(c)** and **FIG. 7(e)**. In the latter case the components are placed in contact so that that fluid flow is possible between these components. The absorbing element **46** is assembled with the cap **50** and an enclosure **48** so that when they are installed into the fluid chamber **44** the tip of the absorbing element **46** is separated from the surface of the substrate **42** to a predefined distance. Substrate **42** is mounted on base **52** such that it can be easily detached once the cells have been collected. Fluid chamber **44** is positioned on the top of the slide **42** which is inserted into the base **52** and is retained by the hooks **30.**

The fluid chamber **44** is releasably attachable to the base as will be described herein after. Different methods of attachment of the fluid chamber **44**to the slide or the base containing the slide may be based on use of adhesives **(****FIGS. 16(a)** to **16(b)** or mechanical retention as shown in **FIGS. 17(a)** to **17(f)** using retaining features **30** when the fluid chamber is pressed in, or **31** when the top of the element **31** is melted and formed a retention feature **32;** or by hooks **34** and **35** aligned by rotation for proper retention of the fluid chamber **44.**

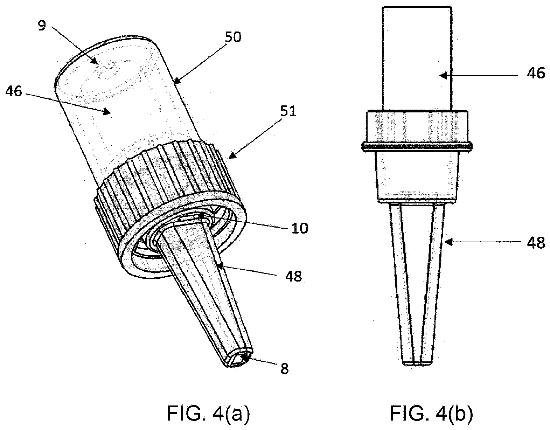

**FIG. 4(a)** is a perspective view of an absorbing element **46** in a plastic enclosure **48** while **FIG. 4(b)** is a front view of the absorbing element **46** in the plastic enclosure **48** of **FIG. 4(a)**. The plastic enclosure **48** can be pressed into the cap **50** or can be retained by the adhesive. The plastic enclosure **48** is equipped with a overfill protection features **10** shown in **FIG 6(a)**. The cap **50** is equipped with riffles **51** on the side surface for ease of use and with a hole **9** on the top of cap **50** to allow release of the differential pressure which may be created during the wicking process.

**FIG. 5(a)** is a perspective view of the tip of the plastic enclosure **48** the absorbing element **46** with a rectangular opening **8** at the tip of the enclosure **48** and **FIG. 5(b)** shows the tip having a circular cross-section opening shown at **8a**. Other shapes of openings are possible as well as long as the cross-sectional area in a combination with other parameters of the absorbing element **46** provides the required flow rate of fluid at the wicking stage of the process.

**FIGS. 6(a)** to **6(e)** show various implementations of controlling the wicking rate during the process of fluid removal after completion of cell sedimentation according to the proposed method. **FIG. 6(a)** shows that controlling of the fluid removal rate can be achieved by selection of the cross-sectional area of the opening **8** in the plastic enclosure **48** which has the rectangular shape as shown in **FIG. 5(a)**. **FIG. 6(b)** shows how this result is achieved by selection of the distance between the plastic enclosure **48** containing an absorbing element **46** and the substrate **42.** It is noted that the result mentioned above can also be achieved by a combination of cross-sectional area and the distance mentioned above. **FIG. 6(c)** shows control of the fluid flow rate by selection of the geometry of the absorbing element **46.**

**FIG. 6(d)** is a photograph showing control of the fluid flow rate can be achieve by using an additional capillary tube **14** attached to the porous material **15** and selection of capillary length and inner diameter. **FIG. 6(e)** shows the capillary **14** attached to the porous material **15** with continuously increasing cross-sectional.

**FIGS. 7(a)** to **7(e)** progressively shows more components related to the absorbing element **46** forming part of the present device. **FIG. 7(a)** shows the tip of the absorbing element **46** placed above the functionalized surface of the solid support **42.** This figure shows the porous flat component **11** in the proximity to the surface used for retention of cells. As it is described further the shape, type of porous material and the distance between the tip of absorbing element and the solid support **42** defines the wicking rate and all these parameters are optimized to reduce the cell loss which can be caused by excessive flow rates. **FIG. 7(b)** shows the absorbing element **46** composed of the flat absorbing tip **11** and cylindrical porous element **82** above absorbing element **11** for increase of the wicking capacity (maximum volume of liquid which can be absorbed by the element **46.** Other configurations of these components are also possible: for example, the element 11 can be made conical with different cross-section; the element 82 referred as a cylindrical element can be made with a square or rectangular cross-section.

The elements **11** and **82** are placed in contact with each other to ensure uninterrupted fluid flow. As a result, these components act from the wicking point of view as a single combined absorbing element 46. **FIG. 7(c)** shows additional increase of the wicking capacity by using the hollow cylindrical porous element **84** into which item **82** is inserted. In a multicomponent absorbing element not only the shape of the elements but also their porosity may be selected iso that the wicking rate and total volume provides required performance. **FIG. 7(d)** shows plastic enclosure for the absorbing element **46** containing plastic cavity **48** and a cap **50** allowing good contact between the parts of the absorbing element **46** and allowing wicking of liquid only through a controlled opening **8** (not shown) in the bottom of plastic cavity **48.** Cap **50** is also equipped with the riffles on the side surface of the cap for ease of use.

**FIG. 7(e)** shows the exploded view of the encapsulated multicomponent absorbing element **90.** Another embodiment of the encapsulated absorbing element **90** contains the plastic cavity **48** the cap **50** and a molded single-component porous absorbing element **46** shown further in **FIG 15(a)** and **FIG 15(b)****.**

**FIG. 8(a)** shows the tip of the absorbing element **46** in the enclosure **48** and a sealing gasket **54** attached to the surface of the solid support **42** or substrate **42** which creates a cavity for the reagents. **FIG. 8(b)** shows an embodiment of a gasket **60** in which a protruding element **62** is added to gasket **54.** The purpose of protruding element **62** is to simplify detachment of the gasket **54** from the slide upon completion of the stages of the process preceding microscopic imaging.

The gasket **54** may have circular, rectangular or square cross sections. They can be affixed to the bottom of fluid chamber **44** by being glued to the bottom of chamber **44** circumferentially extending around the are onto which the target cells sediment. Alternatively, the gasket **54** can be retained in grooves complimentary to the size and shape of the gasket built into the bottom of chamber **44.** Alternatively, the gaskets can be retained on the surface of the solid support **42** and not attached to chamber **44.** It will be appreciated that gaskets may be optional as there are other ways known to those skilled in the art to make a liquid tight seal between chamber **44** and solid support **42.**

**FIGS.8(c)** to **8(f)** is a bottom view of the fluid chamber **44** showing a groove **66 (****FIG. 8(c)**) having a size for accepting gasket **54 (****FIG. 8(d)****).** **FIG. 8(e)** shows the fluid chamber **44** in which an additional groove **68** is added to the bottom of the fluid chamber **44**to accept protruding element **62** of gasket **54** (in which the gasket is absent in **FIG. 8(e). FIG. 8(f)** shows the gasket **54** with its protruding element **62** installed in the fluid chamber **44.**

Once the target cells have been sedimented on the top surface of solid support **42,** the fluid chamber **44** is full of liquid absent the cells. At this point the absorbing element **46,** which is installed into housing **48** along with wicking cap **50,** is inserted into fluid chamber **44** and secured therein by the leak-tight wicking cap **50.** The goal of the absorbing element **46** is to take up the liquid from the fluid chamber **44** in such a way that the cells are not damaged, deformed or released from the surface of support **42** during removal of the liquid. This is achieved by the design parameters primarily of the absorbing element **46,** and secondarily housing **48** and cap **50** which in combination with the design parameters of the housing **48** defines the distance between the absorbing element **46** and its tip and the surface of the support **42.**

With respect to the absorbing element **46,** the fluid absorbing element is designed to have a cross-sectional area, a distribution along an axis of the fluid absorbing element **46** and a porosity in a range to provide control of the rate of absorption of the fluid into the absorbing element **46** along its length and up through the tip spaced from the top surface of the solid support **42,** in order to give a controlled removal of the liquid out of fluid chamber **44.** The mechanical design of the housing**48** is also optimized to assist in the optimum removal of the liquid. The first is the design of housing **48** and cap **50** in order to keep the tip of the absorbing element **46** spaced from the top surface of the solid support **42** to position the tip of the fluid absorbing element at a distance from the surface of the solid support **42** shorter than a predefined distance. The distance that the tip of the absorbing element **46** is positioned from the top surface of the solid substrate **42** onto which the target cells have deposited is important for the following reasons.

Detachment of the target cells attached to the surface of the solid support **42** is mainly caused by the shear stress originated by the fluid flow. The shear stress on the target cells produced by the fluid flow is defined by the derivative of the fluid velocity over the distance. The absolute value of the velocity is defined by the flow rate of the fluid, which is in its turn dependent on the design parameters of the absorbing element **46** and the selected porous material it is made from. An increase of the distance between the solid support **42** and the tip of the absorbing element **46** allows reduction of the shear stress and reduces target cell loss. On the other hand, this also slows down fluid removal and may prevent full removal of fluid from the fluid chamber **44** making the process suboptimal or inefficient. Optimization of all design parameters mentioned above and implementation of flow increasing or accelerating features **17** and **17a** shown in **FIG 10 (a)** and **Fig 10(b)** provides reduced the wicking time for removal of the fluid from fluid chamber **44** and allows fluid removal with a decreased cell loss with acceptable time of fluid removal. The fluid flow provided by accelerating features **17** and **17a** shown in **FIG 10 (a)** and **Fig 10(b)** may be significantly higher compared to the flow provided by the absorbing element **46** through the tip **8** of the enclosure **48,** but as by the time when the absorbing element is introduced into the fluid chamber **44** there are no cells present in the bulk of the fluid and the distance between the feature **17** or **17a** and the support **42** selected to be substantially large there is no cell detachment which is associated with this fluid flow.

In addition to the distance from the top surface of the substrate **46** that the tip is located, the housing **48** may be provided with overflow protection features. **FIG. 9** shows is a view of the absorbing element housing **48** showing overflow protection features **10** which comprise openings in the upper wall of housing **48** to facilitate inflow of fluid into housing **48.** Protection features are located at the top of the housing **48** and they are in close proximity to the part of the absorbing element **46** having a large cross-sectional area. Also, the cross-sectional area of the overflow feature is large and for direct fluid flow to a part of the absorbing element **46** with a large volume of porous material. As a result, the excessive amount of fluid which may initially be present in the fluid chamber **44** will be absorbed and will not overflow the fluid chamber **44.** In addition, these features are located far away from the surface of the solid support **42** where the cells are sedimented at the stage of centrifugation. This guarantees that increase of the flow rate due to the overflow features does not lead to detachment of the cells from the surface and does not lead to cell loss. Example of these overflow feature openings in housing **48** is shown in **FIG 9****.**

**FIG. 10(a)** shows a first embodiment of the absorbent material housing fluid chamber **48** having flow accelerating features **17,** which are elongate slots. **FIG. 10(b)** shows a second embodiment of the absorbent material housing **48** having features **17a,** which are circular holes. The presence of the features **17** or **17a** is to provide an increase of the wicking rate without negative impact on cell retention. This is achieved by placement of features **17** or **17a** further away from the tip of the housing **48** and as a result at a large distance from the surface **42** where the cells are sedimented. Increase of the wicking rate may be required in order to reduce the sample process time.

**FIG. 11(a)** shows a perspective view of the solid support **42** in the form of a glass slide with a functionalized region of the surface of the glass slide being surrounded by gasket **54** with the fluid chamber **44** detached, and **FIG. 11(b)** shows the re-attached fluid chamber **44.** The ability to detach and reattach the fluid chamber **44** allows visual control including microscopy (if required) at different stages of the process. Also, re-attachment of the fluid chamber **44** may be required for cell post-processing after completion of the microscopy stage.

**FIGS. 12(a)** to **12(c)** show the cell retention device at the stage of fluid introduction into fluid chamber **44** in which a first screw cap **68** is removed and the fluid sample flowed into fluid chamber **44,** after which the screw cap **68** is screwed onto fluid chamber **44.** Screw cap **68** is used at the centrifugation stage of the process in order to prevent an accidental leakage of cell containing fluid. This is achieved by using a gasket **70** shown in **FIG 12 (c)** in a form of an O-ring providing a fluid tight connection between the fluid chamber **44** and screw cap **68.** Gasket **70** can have other shapes (e.g., flat) as long as the fluid tight connection is achieved. As noted above, cap **68** is used for the centrifugation stage with the intent to prevent leakage of the fluid containing cells from the fluid chamber **44.** No fluid removal is anticipated during centrifugation step and this allows to keep the cells in fluid preventing damage of the cells which exist in the devices described in other patents or existing on market. After centrifugation is completed cap **68** is replaced with a second screw cap **50** containing the absorbing element **46.** At this stage fluid removal takes place and the fluid is removed in a controlled manner due to proper selection of the porosity of the material used for making and absorbing element **46,** selection of the absorbing element configuration/cross-section and the distance between the tip of the absorbing element and the surface of the solid support **42.** The distance is defined by the design of the enclosure **48** and design of the cap **50.**

Referring to **FIGS. 2(c)** and **2(d)**, device **40** is configured to be releasably held in the centrifuge so that an axis **41** normal to the surface of the solid support **42** and coming through the center of the area onto which the targeted cells deposit onto the top surface of the solid support **42** intersects an axis of rotation **49** in the centrifuge. Orientation of the device in the centrifuge may vary depending on a particular centrifuge used and can be placed in the centrifuge so that the long axis of the solid support 42 is in the plane of rotation (as it is shown in **FIG. 2(b)** or the long axis of the solid support **42** may be perpendicular to the plane of rotation a shown in **FIG. 2(c)****.** Other orientations are possible as well due to axial symmetry of the internal cavity of the fluid chamber **44.**

**FIG. 13** shows the base **52** of the cell retention device with a hole **80** for fluid and cell visualization without detachment of the fluid chamber **44,** and **FIG. 14** shows the solid support **42** of the cell retention device with a section **86** of the surface functionalized for capturing cells and Teflon protected layer **82** around the functionalized area **86.**

**FIG. 15(a)** shows a molded absorbing element **46** with a conical tip **25** with circular cross-section **28,** and **FIG. 15(B)** shows a molded absorbing element **46** with a flat tip with a triangular shape **27** and rectangular cross-section **29.**

**FIGS. 16(a)** to **16(b)** show another embodiment of the proposed device with the absorbing element detached from the assembly **(****FIG 16 (a)****)** and assembled device with the absorbing element in contact with the fluid **(****FIG 16 (b)**). **FIGS. 16(c)** to **16(d)** show another embodiment of the proposed device in which the fluid chamber **44** is glued directly to the surface of slide 42. **FIG 16 (c)** shows the cap **68** attached to the fluid chamber **44** prior to centrifugation step of the process. **FIG 16 (d)** shows the encapsulated absorbing element **46** made of absorbing paper tissue inserted into the fluid chamber **44** for fluid removal after completion of the centrifugation stage.

**FIGS. 17(a)** to **17(f)** show examples of different methods of retaining parts of the assembly. The perspective view in **FIGS. 17(a)** shows the base **52** provided with hooks **30** integrally formed with base **52** along the two opposed long edges which are used to retain fluid chamber assembly **44.** Centrally located in the base **52** is a hole **80.** Located along the two opposed long edges of based **52** are tabs **37** which are used for slide retention.

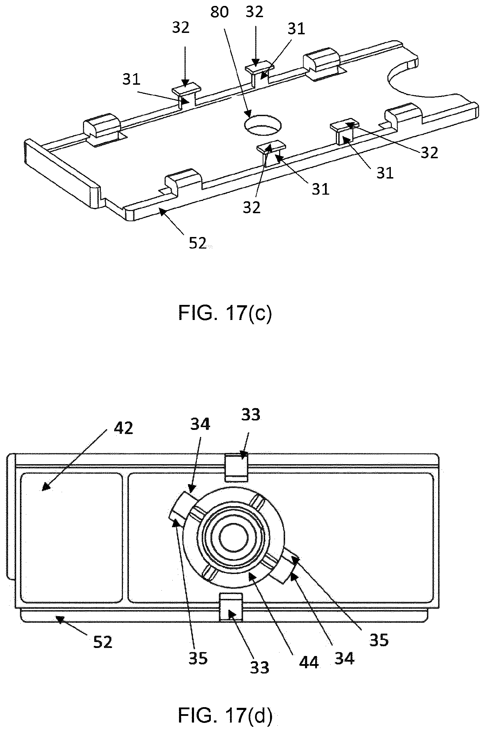

**FIG. 17(b)** shows base **52** using pins **31** integrally formed therewith instead of hooks **30** as in **FIG. 17(a)****.** Pins **31** are compressible using heat so that when fluid chamber **44** is engaged with base **52** the features **32** shown in **FIG. 17(c)** created by compression under heat of pins **31** prevent detachment of the fluid chamber **44** from the base **52.** As the pins **31** are compressed under heat a blob of plastic **32** is created which is larger than an opening through which the pins protrude which keeps the fluid chamber **44** in place.

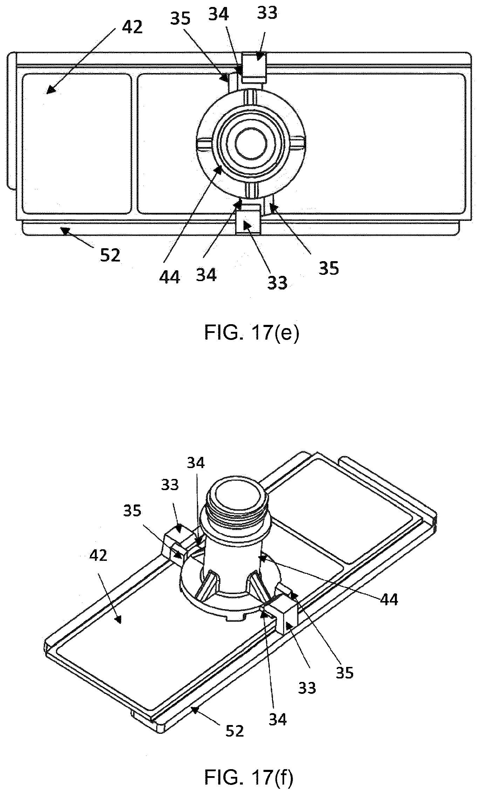

**FIGS. 17(d)** and **17(e)** show another embodiment in which retention of the fluid chamber is achieved retainers **33** engaging with hooks **34** attached to fluid chamber **44** upon rotation of fluid chamber **44.** A stopper **35** is added to the hooks **34** to prevent disengagement of the base **52** with the fluid chamber **44.** **FIG. 17(d)** shows the assembly prior to attachment of the fluid chamber **44** to the base **52** and **FIG. 17(e)** shows the assembled device.

**FIG. 17(f)** shows the fluid chamber assembly **44** locked onto base **52** using the retainers **33** and hooks **34** when the fluid chamber **44** is engaged by sliding the fluid chamber **44** in the direction of the long side od the base **52** contrary to the embodiment shown in **FIG. 17(e)** where engagement is achieved by rotation of the fluid chamber **44.**

**FIG. 17(g)** shows a bottom view of the base **52** with slide **42** retained on the top surface of base **52** with slide **42** including a region **86** which is functionalized with selected agents. Surface functionalization is known to be used for improvement of cells sticking to the surface. Selection of the specific type of functionalization allows increase the retention rate of a particular type of cells which can be used also as a method of enrichment of collected cells or increase of probability of removal of the interfering cells (such as white blood cells) from the surface during the consecutive washing steps following the centrifugation.

**FIG. 17(h)** shows alignment of all components of the assembled device **40** which is important for homogeneous sedimentation of cells and unobscured visualization of the area of the slide where the cells are sedimented.

**FIGS. 18(a)** to **18(f)** inclusive show a series of figures showing the importance of proper alignment of the cell recovery device **40** axis relative to the axis of rotation in a centrifuge. **FIGS. 18(a)** shows a schematic representation of the fluid chamber **44** with the cells in a centrifuge with the axis of the device shifted from the axis of the centrifuge, **FIG. 18(b)** shows force distribution, and **FIG. 18(c)** shows the resulting inhomogeneous distribution of the sedimented cells. **FIG.18(d)** shows plots of calculated displacement of the cells in the direction perpendicular to the axis of rotation from the original position as a function of the initial distance between the cell and the axis of the deice for different displacement of device axis relative to the axis of rotation in a centrifuge for 10 mm, **FIG. 18(e)** shows the same for a displacement of 0.75 mm and **FIG. 18(f)** for a displacement of 0 mm.

**FIG. 19** shows an elevation view of an example of the absorbing element **46** composed of two porous components **82** and **11** and a plastic enclosure **48** for this absorbing element **46** with upper porous component **82** having a volume of 570 microliters and the lower element **11** having a volume of 75 microliters. Total volume of the absorbing element in this embodiment equals to 645 microliters. Assuming that the porosity of the material used for the components of the absorbing element 46 is ~30% the total volume of absorbed liquid can be up to 215 microliters which is slightly higher than the typical volume (around 200 microliters) of the harvest produced by cell separation devices (e.g., Parsortix^{®}). Using porous materials with higher porosities may be advantageous as this will allow for higher wicking rates and lager volumes of absorbed fluid (if required).

**FIG. 20** is a plot of percent cell loss versus wicking rate for two different centrifugation speeds 4400 rpm (circles) and 2200 rpm (triangles).

**FIG. 21** shows percent cell loss as a function of the distance between the surface of the solid support **42** and the absorbing element **46,** forming part of the present cell retention system, without addition of KCI shown in panel (a) and with KCl added to the solution shown in panel (b).

### Optimization of wicking height

Previous optimization of wicking rate was performed on live SK-BR-3 cells that were more adherent and robustly attached than other potential cell types. Working with a less "sticky" cell model: pre-fixed SK-BR-3 cells (ICTs) revealed that previous set height of 0.5 mm was resulting in cell loss during wicking. Therefore, extended distance between microscope slide and wicking tip in effort to decrease cell loss.

It was found that although no cell loss occurred at 1.0 mm wicking height, some changes in the positions of the cells indicated that the cells were still experiencing some form of turbulence, causing them to detach at 1.0 mm. Therefore, for the most gentle process the recommended value for the wicking height was found to be 1.5 mm or more. Wicking height of 2.0 mm opted for instead of 1.5 to provide greater buffer to minimize user to user variability when introducing wicking tip into supernatant.

Optimization of the absorbing material and the distance between the surface of the slide and the tip of the absorbing material allows to select the required flow rate of the fluid, which should be below a threshold defined by shear stress on the cells which may cause detachment of the cells during fluid removal step. Flow rate for two different porous absorbing materials (U200 and U210, Pore Technology, Inc.) were measured for the distance of two mm. The results are shown in **FIGS. 22(a)** and **22(b)****.** Specifically, **FIGS. 22(a)** and **22(b)** show the wicking rate as a function of time for polyethylene porous materials U200 (Pore Technology Inc.) shown in panel **22(a)** and U201 (Pore Technology Inc) shown in panel **22(b).** The distance between the surface of the solid support **42** and the tip of the absorbing element **46** is about 2 mm.

The threshold level for cell detachment was determined experimentally and was equal to 500 uL/s for a selected geometry of the fluid chamber **44** and selected coating of the surface of the solid support **42** (a glass slide). Two mm distance between the solid support **42** and the tip of the absorbing element **46** is appropriate for the absorbing material U200 (see **FIG. 22(a)****)** but this distance should be increased for the material U201 (see **FIG. 22(b)****).**

### Results (pictures of captured cells, evidence of the decreased cell losses)

Pre-labelled SK-BR-3 cells processed through both systems and counted. n = 10 replicates performed for both Cytospin^{®}). and a cell recovery device. Non-parametric Wilcoxon rank sum test performed with a p value of 0.00018. A cell recovery device had an average of 94% ± 6% (SD) cell recovery rate.

**FIG. 23(a)** is a microphotograph of DAPI stained cells spiked into fluid chamber **44** and sedimented by centrifugation using device according to the present method which shows cells retained on the slide surface after centrifugation with consecutive fluid removal. **FIG. 23(b)** is a magnified image (X100) of the microphotograph of **FIG. 23(a). FIGS. 23(a)** and **23(b)** demonstrate improvement of the retention (capture) rate in the proposed method and device compared with the standard in industry Cytospin^{®} method. The improved morphology shows less severe "flattening" of cells and less blebbing, reduced signal intensity of cells on Cytospin^{®} compared to a cell recovery device. Cytoplasmic spreading not seen with a cell recovery device. Staining intensity in a cell recovery device is much higher.

**FIG. 24** shows percent cell capture efficiency for two methods of cell capture: in Cytospin^{®} shown in data set 1 and using the present cell retention method and device shown in data set 2. It is clear from **FIG. 24** that controlled fluid removal rate in combination with other features of the proposed method allows for an increase of the capture rate of cells from approximately 30% (Cytospin^{®} data set in **FIG. 24**) to over 90 - 95% in the proposed device (Harvest Chip data set in **FIG. 24**).

**FIG. 25(a)** shows microphotographs of cells captured using Cytospin^{®} and **FIG. 25(b)** shows microphotographs of cells captured using the cell recovery device of the present cell retention device. The cells captured using Cytospin^{®} can be seen to be damaged while **FIG. 25(b)** clearly shows the present method preserves the morphology of the harvested cells.

**FIG. 26(a)** shows a microphotograph showing the morphology of SK-BR-3 cells which were fixed with lack of fluid around them (referred to as dry fix), while **FIG. 26(b)** shows a microphotograph showing the morphology of SK-BR-3 cells which were fixed in the presence of controlled volume of fluid (referred to as wet fix). The images being obtained using 400x magnification and CK-FITC conjugate stain. As can be seen in **FIGS. 26(a)** and **26(b)****,** control of the residual volume of fluid which is present in the device after the bulk amount of fluid is removed allows for improvement of the retained cell morphology. An additional advantage of controlled volume present in the device at the stage of staining is that the background level is reduced in this case. Fixation with controlled volume of residual fluid produced the lowest background signal compared to Cytospin^{®} or dry fix approach when there is no fluid present (FIG. **26(b)**) or no control of the residual fluid level **(****FIG. 26(a)****).**

It was demonstrated larger overall cell size 15-25 µm diameter is observed in Cytospin^{®} compared to 12-20 µm on the solid support of the cell recovery device (dry fix) and 12-15 µm (wet fix). Also, images obtained with the slides processed using Cytospin^{®} had lower fluorescence signal. Signals with a cell recovery device with controlled volume of residual fluid (Wet fix) exhibited the strongest intensity and dry fix was in-between the Cytospin^{®} and wet fix.

Similar results are obtained with the patient samples processed using Cytospin^{®} and the present cell capture device, see **FIGS. 27(a)** and **27(b)****.** Comparison between Cytospin^{®} and the present device on final morphology: cells retained using Cytospin^{®} in **FIG. 27(a)** appear much larger and more jagged compared to the cells retained using the present device shown in **FIG. 27(b)****.**

EDTA HNV blood spiked with pre-labelled SK-BR-3 cells separated via Parsortix^{®}, pooled, captured, fixed and then stained on either solid support of the cell recovery device or Cytoslide^{™} (Fisher Scientific). (Experiments performed by multiple trained users across multiple instruments! days). Impact of the pipetting variability is reduced (compare STD on Cytospin^{®} and HS). The results are shown in **FIGS. 28** The summary statistics: Cytospin^{®}: 38.8% ± 12.6%, a cell recovery device fix: 96.7% ± 3.3%. As can be seen, significantly better final capture efficiency (counting after staining) on the present a cell recovery device compared to Cytospin^{®}. Most of the cell loss seen in Cytospin^{®} occurred during initial spin down of cells onto slide using the filtercard fluid chamber **44.**

### Post processing of cells

Example of cells post-processing: Lysis of the retained cells with a consecutive molecular analysis is shown in **FIG. 29****,** which shows a histogram of the capture efficiency of EDTA HNV blood spiked with pre-labelled SK-BR-3 cells separated via Parsortix^{®}, pooled, captured, fixed and then stained on either a cell recovery device of the present cell retention device or Cytoslide^{™}. The experiments were performed by multiple trained users across multiple instruments/ days. Impact of the pipetting variability is reduced (compare standard deviation (STD) on Cytospin^{®} and cell recovery device **40**).

Prelabelled SK-BR-3 cell can be seen inside capillary micropipette (image 1) on the surface of the solid support **42** of a cell recovery device followed by suction into microcapillary (image 2) which is subsequently moved to a different location on the slide surface and deposited (image 3). Single cell picking results with the present cell recovery device are shown in **FIG. 30** which shows picking of SK-BR-3 cells deposited onto the solid support with a controlled volume of the residual fluid according to a proposed method. Prelabelled SK-BR-3 cell can be seen inside capillary micropipette (upper panel of **FIG. 30****)** on the surface of the solid support **42** of a cell recovery device followed by suction into microcapillary (middle panel of **FIG. 30**) which is subsequently moved to a different location on the slide surface and deposited (bottom panel of **FIG. 30**).

**FIG. 31** Illustrates that if the parameters are not properly controlled, the cell losses may be high, where **FIG. 31** shows picking of SK-BR-3 cells deposited onto the solid support **42** with a controlled volume of the residual fluid according to a proposed method. The top panel shows a retained cell with a capillary of a cell-picking tool placed above this cell; the middle panel shows the cell being removed from its initial location by the cell-picking tool, and the bottom panel shows the cell dispensed (re-positioned) by the cell-picking tool. The amount of cell loss due to wicking and resuspension steps is minimal when using the cell recovery device apparatus with 2.0 mm wicking height and twopiece wicking cap.

With proper control of the process parameters (wicking rate and volume of residual fluid the cell loss is insignificant even with multiple steps of the process. This is also confirmed by **FIG. 32** which shows % cell recovery at different stages of the process with various protocols as noted in the **FIG. 32****.** Staining of cells directly in the cell recovery device fluid chamber **44** as a post processing step is possible. This would require multiple wicking steps for exchanging reagents within the fluid chamber however this replaces the multiple steps in post slide fixation staining. As can be seen from **FIG. 32** cell loss due to wicking of supernatant and exchange of fluids has been demonstrated to be minimal.

The attainment of good morphology and high recovery is shown in **FIG. 32** which shows percent of cell recovery for multiple consecutive wicking steps (a, b, c) in which the first wicking (a) is done after cell sedimentation, the second wicking (b) is done after resuspension and the third wicking (c) is done after staining, and wherein the test was repeated three times demonstrating that multiple wicking events do not lead to cell losses if the wicking rate is properly selected according to the present method.

Data with Streck SKBR-3 samples counted between each wicking and buffer exchange step shown in **FIG. 32** and the image of the surface with the retained cells stained in the proposed device is shown in **FIG. 34****.**

In-fluid chamber **44** staining of cells with DAPI is shown in **FIG. 33** which is a microphotograph of white blood cells sedimented using the present method disclosed herein and stained with DAPI in which the photograph is taken with the fluid chamber **44** still attached to the solid support demonstrating that the cells can be stained with the fluid chamber **44** attached.

**FIG. 34** shows microphotographs (rows A, B) and a photograph (row C) for the cells sedimented using Cytospin^{®} (column I) and on the solid support **44** of the cell recovery device **40** forming part of the present cell harvesting device (columns I and II) without controlling residual fluid (column II) and with control of residual fluid volume (column III) according to the method disclosed herein. The HNV cells were spiked into a fluid sample placed in the cell recovery device **40** with the following wet fixation method on live EDTA tube cells after staining. The same sample was used for cell sedimentation using Cytospin^{®}. Cytospin^{®} presented the worst morphology (column I, rows A and B) with cytoplasmic spreading and reduced fluorescence intensity as well as increased background. Least amount of morphological alteration was seen in wet fixed a cell recovery device **40** condition (column III). Similar results seen concerning CD45 staining showing reduced cell density in Cytospin^{®} is also of concern.

The following non-limiting and exemplary of protocols for use of device.

### Cell recovery device 40 EDTA Protocol (On-Slide staining)

1. Directly deposit the contents of the Parsortix^{®} harvest into the cell recovery device **40** fluid chamber **44.**
2. Spin down the sample in the StatSpin^{™} CytoFuge^{™} (This is a reference to this device https://www.fishersci.ca/shop/products/statspin-cvtofuge-2-personalcytocentrifuge-system-3/22001 1) (for 2 min at 600 rpm followed by a 2 min spin at 4400 rpm.
3. Screw on the Wicking Cap **50** and allow the device to wick for 5 min.
4. Pipette in 150 µL of ice-cold acetone into the fluid chamber **44** against the tube wall and incubate for 5 min in a freezer (-20 °C) to fix the cells.
5. After fixation, decant the remaining acetone into the appropriate waste container.
6. Detach the fluid chamber **44** from the cell recovery device **40.**
7. Allow any residual acetone to evaporate off and leave a dry slide (10 min) before moving forward to staining or storage.

### Cell recovery device Streck Protocol (On-Slide staining)

1. Directly deposit the contents of the Parsortix^{®} harvest into the cell recovery device **40** fluid chamber **44.**
2. Spin down the sample in the StatSpin^{™} CytoFuge^{™} for 2 min at 600 rpm followed by a 2 min spin at 4400 rpm.
3. Screw on the Wicking Cap and allow the device to wick for 5 min.
4. Prepare a drying buffer consisting of 200 µL solution of 10% Serum (Horse or Fetal Bovine) in 0.083M KCl (i.e., 20 µL Serum + 180 µL KCl solution).
5. Pipette in the 200 µL of drying buffer into the fluid chamber **44** against the tube wall.
6. Spin down the sample in the StatSpin^{™} CytoFuge^{™} for 2 min at 600 rpm.
7. Use a second Wicking Cap **50** to wick off the supernatant.
8. Detach the fluid chamber **44** from the cell recovery device **40.**
9. Disassemble the cell recovery device - pull the PTFE slide out of the Base.
10. Place the PTFE slide with sample onto a hot-plate set to 37°C and allow the liquid remaining on the target area to evaporate for 30 minutes.
11. After confirming that the slide surface has fully dried, proceed to fixation.
12. Using a pipette, introduce 50 µL of ice-cold acetone onto the target area on the slide in a dropwise fashion.
13. Place the Slide in a freezer (-20 °C) for 5 min to fix cells.
14. Decant any residual acetone and allow Slide to dry (10 min) before moving forward to staining or storage.

### Cell recovery device 40 Streck Protocol (In-Solution staining)

**1.** Directly deposit the contents of the Parsortix^{®} harvest into the cell recovery device **40** fluid chamber **44**.
**2.** Spin down the sample in the StatSpin^{™} CytoFuge^{™} for 2 min at 4400 rpm.
3. Screw on the Wicking Cap **50** containing the absorbing element **46** and allow the device to wick for 5 min.
4. Prepare x µL of staining solution (20~50 µL solutions have been tested previously) in permeabilization reagent (e.g., Inside Perm). Preferably, all antibodies will be fluorophore conjugated, thus allowing for a single antibody incubation rather than multiple.
5. Introduce the 50 µL of staining solution into the cell recovery device **40** fluid chamber **44** by pipetting against the wall of the device.
6. Incubate the solution for 45 min at room temperature in the dark.
7. After antibody incubation, gently introduce 200 µL of 1X PBS into the fluid chamber **44** pipetting against the wall.
8. Spin down the sample in the StatSpin^{™} CytoFuge^{™} for 2 min at 4400 rpm.
9. Screw on the Wicking Cap **50** containing the absorbing element **46** and allow the device to wick for 5 min.
10. Repeat the procedure of adding 1X PBS and wicking 2 times to fully wash out any remaining debris.
11. For the final wash prior to cover-slipping, formulate a 200 µL 5% Glycerol solution in Deionized Water.
12. After wicking, use a P1000 pipette to introduce the 5% Glycerol solution into the fluid chamber **44.** Gently mix the solution within the fluid chamber **44** to resuspend the cells in order to ensure an even distribution.
13. Spin the sample down in the StatSpin^{™} CytoFuge^{™} for 2 min at 600 rpm followed by a 2 min spin at 4400 rpm.
14. Screw on the Wicking Cap **50** containing the absorbing element **46** and allow the device to wick for 5 min.
15. Detach the fluid chamber **44** using the Detacher Tool and remove the PTFE Slide containing the sample from the Base.
16. Place the PTFE Slide onto a hot-plate set to 37°C and incubate for 30 minutes. Protect the slides from light to prevent photobleaching.
17. After drying, any remaining glycerol on the target area of the PTFE Slide will still look glassy.
18. Add 1 droplet of mounting media onto the target area and place a coverslip on top. Apply pressure from one edge of the coverslip to prevent air bubbles.

### Cell recovery device 40 protocol - Alternative proteinaceous buffers

1. FBS is labour intensive to prepare (heat inactivate) and difficult to ship.
2. Alternatives (Stabilguard, BSA, Horse Serum, Hank's) explored for equivalent performance to FBS.
3. 10% Horse Serum (same reagent used in blocking step) performed just as well as FBS. Also circumvents need for blocking prior to staining.

### Optimization of fluid removal rate by selection of the absorbing material, the distance between the slide and the tip of the absorbing element and cross-section of the absorbing element.

It was found that porous material by itself absent housing **48** gave an absorption rate that was too fast leading to cell detachment hence using the absorbing element housing **48** was found to be very beneficial in order to limit contact area between liquid medium and porous material. In an embodiment housing **48** can be made of polypropylene. However, it will be appreciated that housing **48** could be made from other materials, including but not limited to polycarbonate, polystyrene, other types of plastic, rubber, glass or metal.

Experiments were conducted using live pre-labelled SK-BR-3 as a model system. These cells were sedimented on the surface of the solid support **42** and then the fluid was removed using a syringe pump with different withdrawal rates. Cell loss was evaluated at each removal rate. As a result, cell loss associated with increasing rates of liquid removal was determined. Tests were conducted on cells sedimented at two different centrifugal forces (265 x g/ 2200 rpm and 1060 x g/ 4400 rpm) to determine maximum allowable fluid removal rate for cells more loosely and strongly adhered to slide surface.

It was determined that for both cells adhered at low centrifugal force (265 x g) and high centrifugal force (1060 x g), the maximum allowable rate of supernatant withdrawal was 500 µL/ min. Cell dissociation from slide surface was observed more drastically at rates higher than 500 µL/min for cells sedimented at 265 x g compared to those sedimented at 1060 x g. To minimize the chances of losing cells during the wicking process, the lower sedimentation force (265 x g) condition was used to determine the maximum allowable wicking rate.

## Claims

1. A cell recovery device (40) for sedimentation and retention of target cells from a fluid sample, comprising:
a) a base (52) configured to releasably hold a solid support (42), the solid support configured to receive cells on a top surface of said solid support;
b) a fluid chamber (44) having a first opening, said fluid chamber providing a liquid tight seal between said top surface and said fluid chamber, said fluid chamber having a second opening for receiving a liquid sample containing the targeted cells being harvested, said first opening defining an area of selected size on the said surface of solid support into which the targeted cells deposit, including a first removable cap (68) configured to close said second opening during centrifugation;
d) said cell recovery device being configured to be received and releasably held in a centrifuge; and
**characterised in that** the cell recovery device further comprises:
e) a fluid absorbing element (46) and an enclosure (48) configured to receive said fluid absorbing element, said enclosure having a bottom opening (8) through which the fluid can enter, said fluid chamber configured to receive said enclosure post centrifugation, said fluid chamber including a second removable cap (50) configured to close said second opening in place of the first removable cap and in combination with the said enclosure prevent positioning of a tip of said fluid absorbing element to a distance from the said surface of said solid support shorter than a predefined distance.

2. The device according to claim 1, wherein said fluid absorbing element (46) has a cross-sectional area, a distribution along an axis of said fluid absorbing element and a porosity in a range to provide control of a rate of absorption of the fluid and the tip of said fluid absorbing element located at the distance from the surface of the solid support (42) such that the target cells settled on the solid support are not detached from the top surface of the solid support by the flow of the fluid being absorbed by the fluid absorbing element.

3. The device according to claims 1 or 2, wherein said fluid absorbing element (46) is a porous material having a porosity in a range from about 1 about 100 microns, and wherein said predefined distance of the tip of the fluid absorbing element above the top surface of solid support (42) is in a range from about 0.1 mm to about 4 mm.

4. The device according to claim 1, wherein said fluid absorbing element (46) has a porosity in a range from about 5 to about 50 microns, and wherein said predefined distance of the tip of the fluid absorbing element above the top surface of solid support is in a range from about 0.1 mm to about 3 mm.

5. The device according to any one of claims 1 to 4, wherein said fluid absorbing element (46) has a porosity in a range from about 10 to about 20 microns, and wherein said predefined distance of the tip of the fluid absorbing element above the top surface of solid support is in a range from about 0.1 mm to about 3 mm.

6. The device according to any one of claims 1 to 5, wherein said area of selected size into which the target cells deposit onto said top surface of said solid support (42) is selected to promote interaction between the target cells and the top surface allowing adherence of the target cells to said top surface of said solid support (42).

7. The device according to any one of claims 1 to 6, wherein said area of selected size into which the target cells deposit onto said top surface of said solid support (42) is modified to promote interaction between the target cells and the top surface to assist adherence of the target cells to said top surface of said solid support.

8. The device according to claim 1, wherein said area (86) of selected size into which the target cells deposit onto said top surface of said solid support (42) is functionalized with agents selected to modify the interaction between the target cells and the top surface to assist adherence of the target cells to said top surface of said solid support.

9. The device according to any one of claims 1 to 10, wherein said fluid absorbing element (46) is made of porous plastic.

10. The device according to any one of claims 1 to 9, wherein the tip of said fluid absorbing element (46) spaced from said top surface is placed at the distance from the top surface of the solid support (42) in the range of about 0.1 mm to about 3 mm.

11. The device according to claim 2, wherein a porosity of said fluid absorbing element (46) and a size of said bottom opening (8) are selected to provide a selected flow rate.

12. The device according to any one of claims 1 to 11, wherein an area on said top surface of said solid support (42) outside of said area of selected size into which the targeted cells deposit onto said top surface of said solid support is coated with a protective coating to restrict the spread of the fluid present in said area of selected size into which the targeted cells deposit onto the rest of the top surface of the said solid support.

13. The device according to any one of claims 1 to 12, wherein said enclosure (48) configured to receive said fluid absorbing element (46) includes overflow features (10) to prevent overflow of fluid caused by insertion of said fluid absorbing element into the fluid chamber (44).

14. The device according to any one of claims 1 to 13, wherein said enclosure (48) configured to receive said fluid absorbing element (46) includes at least one flow accelerating feature (17) to increase the rate of fluid removal without increasing the probability of removal of sedimented cells from the top surface of said solid support.

15. The device according to any one of claims 1 to 14, wherein said solid support (42) is substantially transparent and said base (52) includes an opening (80) aligned with said area of selected size on the said top surface of solid support into which the targeted cells deposit to provide a viewing port for visual viewing of the area of selected size onto which the target cells sediment without the fluid chamber (44) being detached from the solid support.

16. The device according to any one of claims 1 to 15, wherein the fluid tight seal is provided by a gasket (54).

17. The device according to any one of claims 8 to 16, wherein an area of the top surface of the solid support (42) that is not exposed to the fluid sample during cell sedimentation is functionalized with a coating of hydrophobic material (82) different from the functionalized surface area (86), so that when the solid support with the functionalized area is detached from the device and the retained cell area is exposed to small volumes of fluid, said small volumes of fluid contained in the functionalized area are retained by the hydrophobic coating surrounding the area where the target cells are present.

18. A method of cell recovery by sedimentation and retention of target cells from a fluid sample onto a solid support (42), the method comprising:
a) attaching a cell recovery device (40) to the solid support, the cell recovery device having a fluid chamber (44) and a base (52) configured to releasably hold the solid support such that a first opening of the fluid chamber forms a liquid tight seal against a top surface of the solid support, the first opening defining an area of selected size on the top surface of the solid support into which the target cells deposit,
b) placing the fluid sample in the fluid chamber of the cell recovery device through a second opening and attaching a first removable cap (68) to the second opening of the fluid chamber, and
c) subjecting the fluid sample to centrifugation within the fluid chamber to induce sedimentation of the target cells and adhesion of the target cells to the top surface of the solid support,
**characterised in that** the method comprises:
d) post-centrifugation, removing the first removable cap and positioning a fluid absorbing element (46), said fluid absorbing element being in an enclosure (48), within the fluid chamber (44) through the second opening to remove fluid from the fluid chamber, the enclosure (48) configured to receive the fluid absorbing element and having a bottom opening (8) through which the fluid can enter, and attaching a second removable cap (50) to the fluid chamber, the second removable cap configured to close said second opening in place of the first removable cap and in combination with the enclosure prevent positioning of a tip of said fluid absorbing element to a distance from the said top surface of said solid support shorter than a predefined distance.

19. The method according to claim 18, further comprising controlling the flow rate of the fluid being absorbed by the fluid absorbing element (46) such that the sedimented cells are not detached from the top surface of the solid support.

20. The method according to claim 19, wherein the flow rate of the fluid being absorbed by the fluid absorbing element (46) is controlled by the porosity of the material the fluid absorbing element is made of, an area of the bottom opening (8) in the enclosure of the fluid absorbing element and a cross-section of the fluid absorbing element.

21. The method according to claims 18 to 22, further comprising postprocessing of the target cells adhered to the top surface of the solid support (42) by the steps of consecutive introduction and removal of fluid reagents into the fluid chamber (44) so that at each step the flow rate is controlled so that the sedimented cells are not detached and/or lost and the remaining volume of fluid at each consecutive step is controlled to avoid removal of fluid below a predefined volume so that the target cell morphology is not affected by centrifugation in dry conditions.

## Patentansprüche

1. Zellrückgewinnungsvorrichtung (40) zur Sedimentation und Retention von Zielzellen aus einer Fluidprobe, umfassend:
a) eine Basis (52), die konfiguriert ist, um einen festen Träger (42) lösbar zu halten, wobei der feste Träger konfiguriert ist, um Zellen auf einer oberen Oberfläche des festen Trägers aufzunehmen,
b) eine Fluidkammer (44) mit einer ersten Öffnung, wobei die Fluidkammer eine flüssigkeitsdichte Abdichtung zwischen der oberen Oberfläche und der Fluidkammer bereitstellt, wobei die Fluidkammer eine zweite Öffnung zum Aufnehmen einer flüssigen Probe aufweist, welche die geernteten angezielten Zellen enthält, wobei die erste Öffnung einen Bereich von ausgewählter Größe auf der Oberfläche des festen Trägers definiert, in dem sich die angezielten Zellen ablagern, beinhaltend eine erste entfernbare Kappe (68), die konfiguriert ist, um die zweite Öffnung während Zentrifugation zu schließen;
d) wobei die Zellrückgewinnungsvorrichtung konfiguriert ist, um in einer Zentrifuge aufgenommen und lösbar gehalten zu werden; und
**dadurch gekennzeichnet, dass** die Zellrückgewinnungsvorrichtung ferner Folgendes umfasst:
e) ein fluidabsorbierendes Element (46) und ein Gehäuse (48), das konfiguriert ist, um das fluidabsorbierende Element aufzunehmen, wobei das Gehäuse eine Bodenöffnung (8) aufweist, durch die das Fluid eintreten kann, wobei die Fluidkammer konfiguriert ist, um das Gehäuse nach Zentrifugation aufzunehmen, wobei die Fluidkammer eine zweite entfernbare Kappe (50) beinhaltet, die konfiguriert ist, um die zweite Öffnung anstelle der ersten entfernbaren Kappe zu schließen und in Kombination mit dem Gehäuse Positionieren einer Spitze des fluidabsorbierenden Elements in einem Abstand von der Oberfläche des festen Trägers, der kürzer als ein vordefinierter Abstand ist, zu verhindern.

2. Vorrichtung nach Anspruch 1, wobei das fluidabsorbierende Element (46) einen Querschnittsbereich, eine Verteilung entlang einer Achse des fluidabsorbierenden Elements und eine Porosität in einer Spanne aufweist, um Steuerung einer Absorptionsrate des Fluids bereitzustellen und sich die Spitze des fluidabsorbierenden Elements in dem Abstand von der Oberfläche des festen Trägers (42) befindet, sodass die Zielzellen, die sich auf dem festen Träger abgesetzt haben, nicht von der oberen Oberfläche des festen Trägers durch den Fluss des Fluids, das durch das fluidabsorbierende Element absorbiert wird, abgelöst werden.

3. Vorrichtung nach Anspruch 1 oder 2, wobei das fluidabsorbierende Element (46) ein poröses Material mit einer Porosität in einer Spanne von etwa 1 bis 100 Mikrometern ist und wobei der vordefinierte Abstand der Spitze des fluidabsorbierenden Elements über der oberen Oberfläche des festen Trägers (42) in einer Spanne von etwa 0,1 mm bis etwa 4 mm ist.

4. Vorrichtung nach Anspruch 1, wobei das fluidabsorbierende Element (46) eine Porosität in einer Spanne von etwa 5 bis etwa 50 Mikrometern aufweist und wobei der vordefinierte Abstand der Spitze des fluidabsorbierenden Elements über der oberen Oberfläche des festen Trägers in einer Spanne von etwa 0,1 mm bis etwa 3 mm ist.

5. Vorrichtung nach einem der Ansprüche 1 bis 4, wobei das fluidabsorbierende Element (46) eine Porosität in einer Spanne von etwa 10 bis etwa 20 Mikrometern aufweist und wobei der vordefinierte Abstand der Spitze des fluidabsorbierenden Elements über der oberen Oberfläche des festen Trägers in einer Spanne von etwa 0,1 mm bis etwa 3 mm ist.

6. Vorrichtung nach einem der Ansprüche 1 bis 5, wobei der Bereich von ausgewählter Größe, in dem sich die Zielzellen auf der oberen Oberfläche des festen Trägers (42) ablagern, ausgewählt ist, um Wechselwirkung zwischen den Zielzellen und der oberen Oberfläche zu fördern, wodurch Anhaftung der Zielzellen auf der oberen Oberfläche des festen Trägers (42) ermöglicht wird.

7. Vorrichtung nach einem der Ansprüche 1 bis 6, wobei der Bereich von ausgewählter Größe, in dem sich die Zielzellen auf der oberen Oberfläche des festen Trägers (42) ablagern, modifiziert ist, um Wechselwirkung zwischen den Zielzellen und der oberen Oberfläche zu fördern, um Anhaftung der Zielzellen auf der oberen Oberfläche des festen Trägers zu unterstützen.

8. Vorrichtung nach Anspruch 1, wobei der Bereich (86) von ausgewählter Größe, in dem sich die Zielzellen auf der oberen Oberfläche des festen Trägers (42) ablagern, mit Mitteln funktionalisiert ist, die ausgewählt sind, um die Wechselwirkung zwischen den Zielzellen und der oberen Oberfläche zu modifizieren, um Anhaftung der Zielzellen auf der oberen Oberfläche des festen Trägers zu unterstützen.

9. Vorrichtung nach einem der Ansprüche 1 bis 10, wobei das fluidabsorbierende Element (46) aus porösem Kunststoff besteht.

10. Vorrichtung nach einem der Ansprüche 1 bis 9, wobei die Spitze des fluidabsorbierenden Elements (46) beabstandet von der oberen Oberfläche in dem Abstand von der oberen Oberfläche des festen Trägers (42) in der Spanne von etwa 0,1 mm bis etwa 3 mm platziert ist.

11. Vorrichtung nach Anspruch 2, wobei eine Porosität des fluidabsorbierenden Elements (46) und eine Größe der Bodenöffnung (8) ausgewählt sind, um eine ausgewählte Flussrate bereitzustellen.

12. Vorrichtung nach einem der Ansprüche 1 bis 11, wobei ein Bereich auf der oberen Oberfläche des festen Trägers (42) außerhalb des Bereichs von ausgewählter Größe, in dem sich die angezielten Zellen auf der oberen Oberfläche des festen Trägers ablagern, mit einer Schutzschicht beschichtet ist, um die Ausbreitung des Fluids, das in dem Bereich von ausgewählter Größe vorhanden ist, in dem sich die angezielten Zellen ablagern, auf den Rest der oberen Oberfläche des festen Trägers einzuschränken.

13. Vorrichtung nach einem der Ansprüche 1 bis 12, wobei das Gehäuse (48), das konfiguriert ist, um das fluidabsorbierende Element (46) aufzunehmen, Überlaufmerkmale (10) beinhaltet, um Überlaufen von Fluid, das durch Einführung des fluidabsorbierenden Elements in die Fluidkammer (44) verursacht wird, zu verhindern.

14. Vorrichtung nach einem der Ansprüche 1 bis 13, wobei das Gehäuse (48), das konfiguriert ist, um das fluidabsorbierende Element (46) aufzunehmen, zumindest ein Flussbeschleunigungsmerkmal (17) beinhaltet, um die Rate der Fluidentfernung zu erhöhen, ohne die Wahrscheinlichkeit von Entfernung von sedimentierten Zellen von der oberen Oberfläche des festen Trägers zu erhöhen.

15. Vorrichtung nach einem der Ansprüche 1 bis 14, wobei der feste Träger (42) im Wesentlichen transparent ist und die Basis (52) eine Öffnung (80) beinhaltet, die mit dem Bereich von ausgewählter Größe auf der oberen Oberfläche des festen Trägers ausgerichtet ist, in dem sich die angezielten Zellen ablagern, um ein Sichtfenster zur visuellen Betrachtung des Bereichs von ausgewählter Größe bereitzustellen, auf dem die Zielzellen sedimentieren, ohne dass die Fluidkammer (44) von dem festen Träger gelöst wird.

16. Vorrichtung nach einem der Ansprüche 1 bis 15, wobei die fluiddichte Abdichtung durch eine Dichtung (54) bereitgestellt ist.

17. Vorrichtung nach einem der Ansprüche 8 bis 16, wobei ein Bereich der oberen Oberfläche des festen Trägers (42), der nicht der Fluidprobe während Zellsedimentation ausgesetzt ist, mit einer Beschichtung aus anderem hydrophoben Material (82) als dem funktionalisierten Oberflächenbereich (86) funktionalisiert ist, sodass, wenn der feste Träger mit dem funktionalisierten Bereich von der Vorrichtung gelöst ist und der zurückgehaltene Zellbereich kleinen Volumen an Fluid ausgesetzt ist, die kleinen Volumen an Fluid, die in dem funktionalisierten Bereich enthalten sind, durch die hydrophobe Beschichtung, die den Bereich umgibt, in dem die Zielzellen vorhanden sind, zurückgehalten werden.

18. Verfahren zur Zellgewinnung durch Sedimentation und Retention von Zielzellen aus einer Fluidprobe auf einem festen Träger (42), wobei das Verfahren Folgendes umfasst:
a) Anbringen einer Zellrückgewinnungsvorrichtung (40) an dem festen Träger, wobei die Zellrückgewinnungsvorrichtung eine Fluidkammer (44) und eine Basis (52) aufweist, die konfiguriert ist, um den festen Träger lösbar zu halten, sodass eine erste Öffnung der Fluidkammer eine flüssigkeitsdichte Abdichtung gegen eine obere Oberfläche des festen Trägers bildet, wobei die erste Öffnung einen Bereich von ausgewählter Größe auf der oberen Oberfläche des festen Trägers definiert, in dem sich die Zielzellen ablagern,
b) Platzieren der Fluidprobe in der Fluidkammer der Zellrückgewinnungsvorrichtung durch eine zweite Öffnung und Anbringen einer ersten entfernbaren Kappe (68) an der zweiten Öffnung der Fluidkammer, und
c) Unterziehen der Fluidprobe Zentrifugation innerhalb der Fluidkammer, um Sedimentation der Zielzellen und Adhäsion der Zielzellen an der oberen Oberfläche des festen Trägers zu induzieren,
**dadurch gekennzeichnet, dass** das Verfahren Folgendes umfasst:
d) Nachzentrifugieren, Entfernen der ersten entfernbaren Kappe und Positionieren eines fluidabsorbierenden Elements (46), wobei das fluidabsorbierende Element in einem Gehäuse (48) innerhalb der Fluidkammer (44) durch die zweite Öffnung ist, um Fluid aus der Fluidkammer zu entfernen, wobei das Gehäuse (48) konfiguriert ist, um das fluidabsorbierende Element aufzunehmen und eine Bodenöffnung (8) aufweist, durch die das Fluid eintreten kann, und Anbringen einer zweiten entfernbaren Kappe (50) an der Fluidkammer, wobei die zweite entfernbare Kappe konfiguriert ist, um die zweite Öffnung anstelle der ersten entfernbaren Kappe zu schließen und in Kombination mit dem Gehäuse Positionieren einer Spitze des fluidabsorbierenden Elements in einem Abstand von der oberen Oberfläche des festen Trägers, der kürzer als ein vordefinierter Abstand ist, zu verhindern.

19. Verfahren nach Anspruch 18, ferner umfassend Steuern der Flussrate des Fluids, das durch das fluidabsorbierende Element (46) absorbiert wird, sodass die sedimentierten Zellen nicht von der oberen Oberfläche des festen Trägers abgelöst werden.

20. Verfahren nach Anspruch 19, wobei die Flussrate des Fluids, das durch das fluidabsorbierende Element (46) absorbiert wird, durch die Porosität des Materials, aus dem das fluidabsorbierende Element besteht, einen Bereich der Bodenöffnung (8) in dem Gehäuse des fluidabsorbierenden Elements und einen Querschnitt des fluidabsorbierenden Elements gesteuert wird.

21. Verfahren nach Anspruch 18 bis 22, ferner umfassend Nachbearbeiten der Zielzellen, die an der oberen Oberfläche des festen Trägers (42) anhaften, durch die Schritte der aufeinanderfolgenden Einführung und Entfernung von Fluidreagenzien in die Fluidkammer (44), sodass bei jedem Schritt die Flussrate gesteuert wird, sodass die sedimentierten Zellen nicht abgelöst werden und/oder verloren gehen, und das übrige Volumen an Fluid in jedem aufeinanderfolgenden Schritt gesteuert wird, um Entfernung von Fluid unter einem vordefinierten Volumen zu vermeiden, sodass die Zielzellenmorphologie durch Zentrifugation bei trockenen Bedingungen nicht beeinträchtigt wird.

## Revendications

1. Dispositif de récupération de cellules (40) pour la sédimentation et la rétention de cellules cibles à partir d'un échantillon de fluide, comprenant :
a) une base (52) conçue pour maintenir de manière amovible un support solide (42), le support solide étant conçu pour recevoir des cellules sur une surface supérieure dudit support solide ;
b) une chambre à fluide (44) comportant une première ouverture, ladite chambre à fluide assurant un joint étanche aux liquides entre ladite surface supérieure et ladite chambre à fluide, ladite chambre à fluide comportant une seconde ouverture destinée à recevoir un échantillon de liquide contenant les cellules ciblées en cours de culture, ladite première ouverture définissant une zone de taille choisie sur ladite surface de support solide dans laquelle les cellules ciblées se déposent, comprenant un premier capuchon amovible (68) conçu pour fermer ladite seconde ouverture pendant la centrifugation ;
d) ledit dispositif de récupération de cellules étant conçu pour être reçu et maintenu de manière amovible dans une centrifugeuse ; et
**caractérisé en ce que** le dispositif de récupération de cellules comprend en outre :
e) un élément d'absorption de fluides (46) et une enceinte (48) conçue pour recevoir ledit élément d'absorption de fluides, ladite enceinte comportant une ouverture inférieure (8) à travers laquelle le fluide peut entrer, ladite chambre à fluide étant conçue pour recevoir ladite enceinte après centrifugation, ladite chambre à fluide comprenant un second capuchon amovible (50) conçu pour fermer ladite seconde ouverture à la place du premier capuchon amovible et, en combinaison avec ladite enceinte, empêcher le positionnement d'une pointe dudit élément d'absorption de fluides à une certaine distance de ladite surface dudit support solide plus courte que la distance prédéfinie.

2. Dispositif selon la revendication 1, ledit élément d'absorption de fluides (46) comportant une surface de section transversale, une distribution le long d'un axe dudit élément d'absorption de fluides et une porosité dans une plage permettant d'assurer le contrôle du taux d'absorption du fluide et la pointe dudit élément d'absorption de fluides étant située à une distance de la surface du support solide (42) de sorte que les cellules cibles sédimentées sur le support solide ne se détachent pas de la surface supérieure du support solide par l'écoulement du fluide absorbé par l'élément d'absorption de fluides.

3. Dispositif selon la revendication 1 ou 2, ledit élément d'absorption de fluides (46) étant un matériau poreux comportant une porosité comprise dans la plage allant d'environ 1 à environ 100 microns, et ladite distance prédéfinie de la pointe de l'élément d'absorption de fluides au-dessus de la surface supérieure du support solide (42) étant comprise dans la plage allant d'environ 0,1 mm à environ 4 mm.

4. Dispositif selon la revendication 1, ledit élément d'absorption de fluides (46) comportant une porosité comprise dans la plage allant d'environ 5 à environ 50 microns, et ladite distance prédéfinie de la pointe de l'élément d'absorption de fluides au-dessus de la surface supérieure du support solide étant comprise dans la plage allant d'environ 0,1 mm à environ 3 mm.

5. Dispositif selon l'une quelconque des revendications 1 à 4, ledit élément d'absorption de fluides (46) comportant une porosité comprise dans la plage allant d'environ 10 à environ 20 microns, et ladite distance prédéfinie de la pointe de l'élément d'absorption de fluides au-dessus de la surface supérieure du support solide étant comprise dans la plage allant d'environ 0,1 mm à environ 3 mm.

6. Dispositif selon l'une quelconque des revendications 1 à 5, ladite zone de taille choisie dans laquelle les cellules cibles se déposent sur ladite surface supérieure dudit support solide (42) étant choisie de manière à favoriser l'interaction entre les cellules cibles et la surface supérieure permettant l'adhérence des cellules cibles à ladite surface supérieure dudit support solide (42).

7. Dispositif selon l'une quelconque des revendications 1 à 6, ladite zone de taille choisie dans laquelle les cellules cibles se déposent sur ladite surface supérieure dudit support solide (42) étant modifiée de manière à favoriser l'interaction entre les cellules cibles et la surface supérieure afin de faciliter l'adhérence des cellules cibles à ladite surface supérieure dudit support solide.

8. Dispositif selon la revendication 1, ladite zone (86) de taille choisie dans laquelle les cellules cibles se déposent sur ladite surface supérieure dudit support solide (42) étant fonctionnalisée avec des agents choisis de manière à modifier l'interaction entre les cellules cibles et la surface supérieure afin de faciliter l'adhérence des cellules cibles à ladite surface supérieure dudit support solide.

9. Dispositif selon l'une quelconque des revendications 1 à 10, ledit élément d'absorption de fluides (46) étant constitué de plastique poreux.

10. Dispositif selon l'une quelconque des revendications 1 à 9, ladite pointe dudit élément d'absorption de fluides (46) espacée de ladite surface supérieure étant placée à une distance de la surface supérieure du support solide (42) comprise dans la plage allant d'environ 0, 1 mm à environ 3 mm.

11. Dispositif selon la revendication 2, la porosité dudit élément d'absorption de fluides (46) et la taille de ladite ouverture inférieure (8) étant choisies de manière à assurer un débit choisi.

12. Dispositif selon l'une quelconque des revendications 1 à 11, une zone sur ladite surface supérieure dudit support solide (42) à l'extérieur de ladite zone de taille choisie dans laquelle les cellules ciblées se déposent sur ladite surface supérieure dudit support solide étant recouverte d'un revêtement protecteur destiné à limiter la propagation du fluide présent dans ladite zone de taille choisie dans laquelle les cellules ciblées se déposent sur le reste de la surface supérieure dudit support solide.

13. Dispositif selon l'une quelconque des revendications 1 à 12, ladite enceinte (48) conçue pour recevoir ledit élément d'absorption de fluides (46) comprenant des éléments de débordement (10) destinés à empêcher un débordement de fluide provoqué par l'insertion dudit élément d'absorption de fluides dans la chambre à fluide (44).

14. Dispositif selon l'une quelconque des revendications 1 à 13, ladite enceinte (48) conçue pour recevoir ledit élément d'absorption de fluides (46) comprenant au moins un élément d'accélération de flux (17) destiné à augmenter la vitesse d'élimination du fluide sans augmenter la probabilité d'élimination des cellules sédimentées de la surface supérieure dudit support solide.

15. Dispositif selon l'une quelconque des revendications 1 à 14, ledit support solide (42) étant sensiblement transparent et ladite base (52) comprenant une ouverture (80) alignée avec ladite zone de taille choisie sur ladite surface supérieure du support solide dans laquelle les cellules ciblées se déposent pour fournir un orifice de visualisation permettant de voir visuellement la zone de taille choisie sur laquelle les cellules cibles sédimentent sans que la chambre à fluide (44) ne soit détachée du support solide.

16. Dispositif selon l'une quelconque des revendications 1 à 15, ledit joint étanche aux fluides étant assuré par une garniture d'étanchéité (54).

17. Dispositif selon l'une quelconque des revendications 8 à 16, une zone de la surface supérieure du support solide (42) qui n'est pas exposée à l'échantillon de fluide pendant la sédimentation des cellules étant fonctionnalisée avec un revêtement de matériau hydrophobe (82) différent de la zone de surface fonctionnalisée (86), de sorte que lorsque le support solide avec la zone fonctionnalisée est détaché du dispositif et que la zone de cellules retenues est exposée à de petits volumes de fluide, lesdits petits volumes de fluide contenus dans la zone fonctionnalisée soient retenus par le revêtement hydrophobe entourant la zone dans laquelle les cellules cibles sont présentes.

18. Procédé de récupération de cellules par sédimentation et rétention de cellules cibles à partir d'un échantillon de fluide sur un support solide (42), le procédé comprenant :
a) la fixation d'un dispositif de récupération de cellules (40) au support solide, le dispositif de récupération de cellules comportant une chambre à fluide (44) et une base (52) conçue pour maintenir de manière amovible le support solide de sorte qu'une première ouverture de la chambre à fluide forme un joint étanche aux liquides contre une surface supérieure du support solide, la première ouverture définissant une zone de taille choisie sur la surface supérieure du support solide dans laquelle les cellules cibles se déposent,
b) le placement de l'échantillon de fluide dans la chambre à fluide du dispositif de récupération de cellules à travers une seconde ouverture et la fixation d'un premier capuchon amovible (68) à la seconde ouverture de la chambre à fluide, et
c) la soumission de l'échantillon de fluide à une centrifugation dans la chambre à fluide de manière à induire la sédimentation des cellules cibles et l'adhérence des cellules cibles à la surface supérieure du support solide,
**caractérisé en ce que** le procédé comprend :
d) après la centrifugation, le retrait du premier capuchon amovible et le positionnement d'un élément d'absorption de fluides (46), ledit élément d'absorption de fluides se trouvant dans une enceinte (48), à l'intérieur de la chambre à fluide (44) à travers la seconde ouverture pour éliminer le fluide de la chambre à fluide, l'enceinte (48) étant conçue pour recevoir l'élément d'absorption de fluides et comportant une ouverture inférieure (8) à travers laquelle le fluide peut entrer, et la fixation d'un second capuchon amovible (50) à la chambre à fluide, le second capuchon amovible étant conçu pour fermer ladite seconde ouverture à la place du premier capuchon amovible et en combinaison avec l'enceinte empêcher le positionnement d'une pointe dudit élément d'absorption de fluides à une distance de ladite surface supérieure dudit support solide inférieure à une distance prédéfinie.

19. Procédé selon la revendication 18, comprenant en outre le contrôle du débit du fluide absorbé par l'élément d'absorption de fluides (46) de sorte que les cellules sédimentées ne soient pas détachées de la surface supérieure du support solide.

20. Procédé selon la revendication 19, ledit débit du fluide absorbé par l'élément d'absorption de fluides (46) étant contrôlé par la porosité du matériau dont est constitué l'élément d'absorption de fluides, une zone de l'ouverture inférieure (8) dans l'enceinte de l'élément d'absorption de fluide et une section transversale de l'élément d'absorption de fluides.

21. Procédé selon les revendications 18 à 22, comprenant en outre le post-traitement des cellules cibles adhérant à la surface supérieure du support solide (42) par les étapes d'introduction et d'élimination consécutives de réactifs fluides dans la chambre à fluide (44) de sorte qu'à chaque étape, le débit soit contrôlé de manière à ce que les cellules sédimentées ne soient pas détachées et/ou perdues et que le volume restant de fluide à chaque étape consécutive soit contrôlé pour éviter l'élimination de fluide en dessous d'un volume prédéfini de sorte que la morphologie des cellules cibles ne soit pas affectée par la centrifugation dans des conditions sèches.
